(19) Europäisches Patentamt
European Patent Office
Office européen des brevets

(11) **EP 2 588 639 B1**

(12) # FASCICULE DE BREVET EUROPEEN

(45) Date de publication et mention
de la délivrance du brevet:
**07.09.2016 Bulletin 2016/36**

(21) Numéro de dépôt: **11741624.8**

(22) Date de dépôt: **01.07.2011**

(51) Int Cl.:
***C23C 14/48*** *(2006.01)*

(86) Numéro de dépôt international:
**PCT/FR2011/051544**

(87) Numéro de publication internationale:
**WO 2012/001325 (05.01.2012 Gazette 2012/01)**

(54) **PROCEDE DE TRAITEMENT DE SURFACE D'UN DISPOSITIF DE DISTRIBUTION DE PRODUIT FLUIDE.**

VERFAHREN ZUR OBERFLÄCHENBEARBEITUNG EINER VORRICHTUNG ZUR AUSGABE EINES FLUIDPRODUKTS

METHOD FOR THE SURFACE TREATMENT OF A FLUID PRODUCT DISPENSING DEVICE

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **16.07.2010 FR 1002989**
**02.07.2010 FR 1055343**

(43) Date de publication de la demande:
**08.05.2013 Bulletin 2013/19**

(73) Titulaire: **Aptar France SAS**
**27110 Le Neubourg (FR)**

(72) Inventeurs:
• **BRUNA, Pascal**
**F-76300 Sotteville Les Rouen (FR)**
• **BUSARDO, Denis**
**F-14510 Gonneville Sur Mer (FR)**

(74) Mandataire: **CAPRI**
**33, rue de Naples**
**75008 Paris (FR)**

(56) Documents cités:
WO-A1-2010/100384   WO-A2-2008/141141
FR-A1- 2 917 753   GB-A- 2 071 673
US-A- 5 492 763   US-A- 5 520 664

• **DATABASE WPI Week 200770 Thomson Scientific, London, GB; AN 2007-747865 XP002630000, -& KR 2006 122 073 A (YANG W D) 30 novembre 2006 (2006-11-30)**
• **RAGHUVIR SINGH ET AL: "Corrosion degradation and prevention by surface modification of biometallic materials", JOURNAL OF MATERIALS SCIENCE: MATERIALS IN MEDICINE, KLUWER ACADEMIC PUBLISHERS, BO, vol. 18, no. 5, 2 décembre 2006 (2006-12-02), pages 725-751, XP019503689, ISSN: 1573-4838**
• **LUKE HANLEY ET AL: "The growth and modification of materials via ion surface processing", SURFACE SCIENCE, NORTH-HOLLAND PUBLISHING CO, AMSTERDAM, NL, vol. 500, no. 1-3, 10 mars 2002 (2002-03-10), pages 500-522, XP002622947, ISSN: 0039-6028, DOI: 10.1016/S0039-6028(01)01528-X [extrait le 2001-09-15]**
• **RAO GOPAL R ET AL: "Effects of sequential He<+> and Ar<+> implantation on surface properties of polymers", JOURNAL OF MATERIALS RESEARCH, MATERIALS RESEARCH SOCIETY, WARRENDALE, PA, US, vol. 11, no. 10, 1 octobre 1996 (1996-10-01), pages 2661-2667, XP009123709, ISSN: 0884-2914**

• LEE E H ET AL: "Ion Beam application for improved polymer surface properties", NUCLEAR INSTRUMENTS & METHODS IN PHYSICS RESEARCH, SECTION - B:BEAM INTERACTIONS WITH MATERIALS AND ATOMS, ELSEVIER, AMSTERDAM, NL, vol. 74, no. 1-2, 2 avril 1993 (1993-04-02), pages 326-330, XP002622948, ISSN: 0168-583X, DOI: 10.1016/0168-583X(93)95070-L [extrait le 2002-10-15]

**Description**

La présente invention concerne un procédé de traitement de surface pour des dispositifs de distribution de produits fluides.

Les dispositifs de distribution de produits fluides sont bien connus. Ils comportent généralement un réservoir, un organe de distribution, tel qu'une pompe ou une valve, et une tête de distribution pourvue d'un orifice de distribution. En particulier dans le domaine pharmaceutique, les risques de contamination du produit fluide à distribuer peuvent être critiques, notamment lorsque ces produits sont dépourvus de conservateurs. Ainsi, des dispositifs de distribution par voie nasale ou orale peuvent subir des contaminations bactériennes. Ces contaminations peuvent se produire notamment à l'intérieur du réservoir dans lequel le produit fluide est stocké, notamment par pénétration de bactéries à travers l'orifice de distribution, ou à l'extérieur du réservoir au contact du patient, notamment autour de l'orifice de distribution. L'apparition et/ou la multiplication de bactéries sur une surface donnée dépend pour grande partie de la présence d'un biofilm qui se forme sur ladite surface. Pour limiter ces risques de contamination bactérienne, il a été proposé de filtrer l'air d'éventation ou d'utiliser des pompes sans reprises d'air. On peut aussi prévoir un obturateur directement en amont de l'orifice de distribution, qui empêche la prolifération de contamination bactérienne vers le réservoir entre deux utilisations du dispositif. Ces solutions sont toutefois insuffisantes pour les surfaces externes, par exemple les parois en contact avec l'intérieur des narines lors d'une distribution nasale. Elles sont aussi insuffisantes pour les surfaces internes, notamment pendant la phase de distribution, lorsqu'un éventuel obturateur est en position ouverte. Pour davantage limiter les risques de contamination bactérienne à l'intérieur et à l'extérieur des dispositifs, il a été proposé de revêtir certaines surfaces internes et/ou externes, destinées à venir en contact avec le produit fluide à distribuer, avec des produits bactéricides et/ou bactériostatiques, par exemple des couches contenant des ions d'argent. Les documents suivants décrivent diverses solutions de l'art antérieur : EP0473892, EP0580460, EP0831972, US6227413, EP1169241, DE2830977, US5154325, EP0644785, US5433343, EP0889757. Ces solutions ne sont toutefois pas satisfaisantes. Ainsi, l'efficacité et la durabilité de ces revêtements sont discutables, et ils ne permettent généralement pas de remplir les exigences réglementaires relatives aux tests bactériologiques pour des distributeurs de médicaments sans conservateurs.

La présente invention a ainsi pour but de proposer un procédé de traitement de surface qui ne reproduit pas les inconvénients susmentionnés.

En particulier, la présente invention a pour but de fournir un procédé de traitement de surface qui soit efficace, durable, non polluant, qui n'interagisse pas avec le produit fluide et qui soit simple à réaliser.

Selon un aspect particulier, l'invention propose un procédé de greffage de monomères en couche profonde dans un matériau organique par un faisceau d'ions.

L'invention vise notamment à réduire la prolifération des bactéries, notamment sur des embouts de sprays nasaux entre deux prises de médicament.

On entend par « organique » un matériau constitué d'atomes de carbone liés entre eux ou à d'autres atomes par des liaisons covalentes. On range dans cette catégorie les matériaux appartenant par exemple à la famille des polymères, des élastomères ou des résines. Ces matériaux organiques ont pour spécificité d'être généralement des isolants électriques et sujets à la production de radicaux libres sous l'effet de rayonnements ionisants parmi lesquels on compte les rayons UV, X, y, les faisceaux d'électrons, les faisceaux d'ions.

Par exemple une liaison covalente de type C=C donne sous l'effet d'un rayonnement ionisant deux radicaux libres notés (.) situés chacun sur un atome de carbone(.C-C.) et capables de se recombiner avec d'autres molécules (par exemple $O_2$) dans des réactions radicalaires caractérisées par trois étapes, la première d'initiation, la seconde de propagation et la troisième d'inhibition.

On entend par monomère une molécule simple utilisée pour la synthèse des polymères. Pour être greffables au matériau organique, ces monomères doivent présenter des insaturations (par exemple une double liaison) capables de réagir avec les radicaux libres engendrés dans le matériau organique par le rayonnement ionisant.

L'exposition d'un matériau polymère à des rayonnements ionisants de type bombardement électronique ou rayons gamma créé des radicaux libres (réaction d'ionisation) qui peuvent soit se recombiner entre eux par des réactions dites de réticulation en créant de nouvelles liaisons covalentes entre atomes du matériau organique, soit de permettre le greffage de monomères venues de l'extérieur avec des atomes du matériau organique. Les radicaux libres réagissent avec des monomères présentant une insaturation de type fonction vinylique ou acrylique. Les rayonnements ionisants par bombardement électronique ou rayons gamma et les installations d'irradiation associées, permettent le greffage de supports sous des formes très différentes : films, surfaces textiles, granulés compoundés, dispositifs médicaux par exemple. Un monomère porteur d'une insaturation greffable de type vinylique, allylique ou acrylique peut se fixer sur une chaîne carbonée sous l'effet de rayonnements ionisants. Selon les autres fonctions chimiques (ou ligands) portées par le monomère, des caractéristiques particulières peuvent ainsi être conférées de manière permanente au matériau support : propriété antiseptiques, échangeurs ioniques, promoteurs d'adhésion ...

Les procédés de greffage par bombardement électronique ou rayons gamma présentent toutefois des inconvénients liés aux moyens de production des particules ionisantes et à leur portée, ce qui a pour effet de limiter fortement leur usage.

Les installations produisant les rayons gamma sont extrêmement lourdes à gérer aussi bien sur le plan technique que sur le plan de la sécurité. Elles consistent en une source radioactive de cobalt-60, sous la forme de crayons, confinée dans une casemate en béton avec des murs de 2 m d'épaisseur. La casemate abrite également une piscine de stockage des panneaux de source, destinée à la protection biologique lorsque la source est en position de « repos ». En position de « travail », un convoyeur aérien, porteur de containers (encore appelés balancelles), assure la circulation des produits à traiter autour de la source suspendue dans la cellule, ainsi que le transfert des produits entre l'intérieur et l'extérieur de la casemate. La géométrie à chicane de cette dernière assure le confinement du rayonnement, tout en permettant le défilement continu des produits. La puissance de la source peut atteindre plusieurs millions de curie.

Les installations produisant les faisceaux électroniques sont tout aussi lourdes dans leur mise en oeuvre. Il faut en effet prévoir des systèmes de blindages épais pour arrêter la production intense de rayonnement X engendrée par la décélération des électrons dans le matériau. D'autre part, les faisceaux électroniques peuvent par l'accumulation de charges électrostatiques au coeur d'un matériau organique isolant, provoquer son éclatement.

Un autre inconvénient cette fois-ci physique, est lié au pouvoir de pénétration trop élevé des rayonnements gamma (plusieurs mètres) et des électrons (plusieurs mm). Ces pouvoirs de pénétration ne sont pas adaptés pour un traitement ou l'on cherche à traiter la surface sans modifier les propriétés massiques du matériau organique. Il n'est pas souhaitable en effet, qu'un élastomère perde ses propriétés d'élasticité massique et gagne en rigidité au point de ne plus pouvoir par exemple épouser une surface galbée (par exemple un essuie glace).

Il existe un autre procédé de greffage agissant cette fois-ci en extrême surface mettant en oeuvre un plasma froid. Les plasmas froids sont des milieux ionisés obtenus par l'excitation d'un gaz (en général sous un vide primaire) sous l'effet d'une décharge électrique : les plasmas radiofréquence (kHz au MHz) et micro-onde (2.45 GHz) sont les plus couramment utilisés. On obtient ainsi un mélange constitué de molécules neutres (majoritaires) d'ions (négatifs et positifs), d'électrons, d'espèces radicalaires (chimiquement très actives) et d'espèces excitées. Ces plasmas sont dits "froids" car ce sont des milieux hors équilibre thermodynamique où l'énergie est captée essentiellement par les électrons mais où la température "macroscopique" du gaz reste voisine de la température ambiante. Les électrons émis par l'électrode entrent en collision avec les molécules de gaz et les activent. Il se produit alors une ionisation ou une dissociation avec création de radicaux. Ces espèces excitées vont diffuser dans l'enceinte du réacteur et en particulier arriver à la surface du substrat. Là peuvent intervenir plusieurs types de réactions de surface : implantation à de très faibles énergies (quelques nm), transfert d'énergie, création de liaisons ou destruction de liaisons. Selon le type de réaction intervenant à la surface on pourra avoir activation de la surface, croissance d'une couche, ou gravure. Le greffage chimique par plasmas froid consiste à travailler avec des gaz tels que l'oxygène, l'azote, l'air, l'ammoniac ou le tétrafluorocarbone dont les espèces actives vont réagir chimiquement sur les chaînes macromoléculaires du polymère pour conduire à la formation de liaisons covalentes (C-O, CN, C-F,...) caractéristiques du gaz de traitement. Ce type de traitement affecte uniquement les premiers nanomètres de la surface exposée au plasma. La surface d'un polymère ainsi activée peut ensuite être mise en contact avec des molécules biocompatibles spécifiques (héparine, phospholipides, etc) pour les fixer par liaisons chimiques. Généralement le greffage chimique est réalisé en plaçant le matériau à traiter en dehors de la zone de création de la décharge (post-décharge). En raison de l'extrême faiblesse des épaisseurs greffées le traitement a une efficacité limitée dans le temps. Il est également sensible aux conditions d'usage (usure, frottement, abrasion) qui peuvent entrainer très tôt sa disparition.

Il en résulte un besoin de méthode de greffage en couche profonde d'un matériau organique, de préférence selon des méthodes facilement industrialisables, de manière à pouvoir offrir de telles matériaux organiques en quantité significative et à des coûts raisonnables.

L'invention a ainsi aussi pour but d'offrir une méthode greffage en couche profonde de matériau organique peu onéreuse et permettant de traiter des surfaces répondant aux besoins de nombreuses applications.

L'invention propose ainsi un procédé de greffage en couche profonde par un faisceau d'ions d'un matériau organique qui comprend deux étapes:

a) un bombardement ionique où:

- les ions du faisceau d'ions sont sélectionnés parmi les ions des éléments de la liste constituée de l'hélium (He), du bore (B), du carbone (C), de l'azote (N), de l'oxygène (O), du néon (Ne), de l'argon (Ar), du krypton (Kr), du xénon (Xe) ;
- la tension d'accélération des ions est supérieure ou égale à 10 kV et inférieure ou égale à 1000 kV ;
- la température du matériau organique est inférieure ou égale à la température de fusion;
- on choisit la dose d'ions par unité de surface dans une plage comprise entre $10^{12}$ ions/cm$^2$ et $10^{18}$ ions/cm$^2$ de manière à créer par bombardement ionique une couche constituant un réservoir de radicaux libres permettant lors d'une seconde étape le greffage de monomères. Ce réservoir de radicaux libres se caractérise par une couche superficielle d'une épaisseur de l'ordre de quelques microns. Ce réservoir de radicaux libres peut éventuellement être séparé du milieu ambiant par une couche en extrême surface totalement réticulée par le bombardement ionique et constituée essentiellement de carbone amorphe. Cette couche de carbone amorphe en extrême surface qui est par nature moins réactive, a un effet stabilisant sur le réservoir de radicaux libre vis à vis du milieu ambiant et permet d'augmenter la dureté superficielle du matériau organique.

b) une étape de greffage de monomères consistant à faire diffuser des monomères de la surface vers le réservoir de radicaux libres à une température de diffusion choisie judicieusement pour les greffer aux molécules présentes dans ledit réservoir. La température de diffusion doit être choisie de manière :

- à activer les radicaux libres présents dans les épaisseurs traitées (couche stabilisante + réservoir de radicaux libres) ;
- accélérer le processus de diffusion des monomères de la surface au travers de la couche stabilisante vers le réservoir de radicaux libres ;
- à accélérer les mécanismes radicalaires aboutissant au greffage des monomères aux molécules présentes dans le réservoir ;
- à garantir que les propriétés du matériau organique ne sont pas altérées lors du retour à la température ambiante.

Selon un mode de réalisation la température de transition vitreuse Tg semble la plus indiquée. Selon un autre mode de réalisation on se réserve la possibilité d'explorer des températures intermédiaires comprises entre la température de transition vitreuse Tg et la température de fusion assortie de précautions quant aux conditions de refroidissement pour retrouver les propriétés du matériau organique d'origine. Enfin selon un troisième mode on se réserve la possibilité d'explorer des températures comprises entre la température ambiante et la température de transition vitreuse si la densité et la réactivité des radicaux libres, la vitesse de diffusion des monomères sont suffisamment importantes pour réduire fortement les temps de greffage. Le choix de la température de diffusion dépend énormément de la nature du matériau organique et du monomère greffable.

Le choix des ions et des conditions de bombardement de ces ions permet d'identifier avantageusement un réservoir de radicaux libres d'une densité optimale pour greffer en couche profonde dans une épaisseur de l'ordre du micron, une densité élevée de monomères dont les propriétés peuvent être hydrophobes, hydrophiles, anti bactériens ou encore conducteur. On est capable ainsi de créer des barrières épaisses et très efficaces de nature hydrofuge, hydrophile, antibactérienne ou encore conducteur. On citera pour exemple :

- Monomères hydrophiles : acide acrylique
- Monomères hydrophobes : 2-(perfluoro-3-methylbutyl) ethylmethacrylate3-(perfluoro-3-methylbutyl)-2-hydroxy propyl methacrylate
- Monomère antibactériens : bromure ou chlorure de diméthyloctyl ammonium éthylméthacrylate, complexe méthacrylate d'éthylène glycol phosphaté - ion argent.

Les inventeurs ont pu constater que les plages choisies selon l'invention de tension d'accélération et de dose d'ions par unité de surface permettent de sélectionner des conditions expérimentales optimales où le greffage en couche profonde est possible grâce à un bombardement ionique traitant des épaisseurs de l'ordre du micron.

En outre, ils ont pu constater que le procédé selon l'invention peut être mis en oeuvre « à froid », notamment à température ambiante et qu'il convient que la température du matériau organique demeure inférieure ou égale à la température de fusion durant la mise en oeuvre du procédé. On peut ainsi éviter avantageusement que le matériau organique subisse une modification physico-chimique ou mécanique ou que les radicaux libres se recombinent entre eux.

Le procédé selon l'invention présente l'avantage modifier les caractéristiques superficielles du matériau organique dans une épaisseur de l'ordre du micron sans altérer les propriétés de masse.

Le choix de la dose d'ions par unité de surface dans la plage de dose selon l'invention peut résulter d'une étape préalable d'étalonnage où on bombarde avec un des ions parmi He, B, C, N, O, Ne, Ar, Kr, Xe, un échantillon constitué du matériau organique envisagé. Le bombardement de ce matériau organique peut s'effectuer dans différentes zones du matériau avec une pluralité de doses d'ions, dans la plage selon l'invention, et on mesure en condition ambiante l'évolution temporelle de la résistivité superficielle des zones traitées de manière à identifier après une durée liée à la diffusion de l'oxygène dans le matériau organique, un saut résistif caractéristique de l'oxydation très rapide du réservoir de radicaux libre sous jacent à la surface.

Les inventeurs ont pu constater que la hauteur du saut résistif donne une estimation de la densité de radicaux libres présents dans le réservoir et que le choix d'une dose pour un matériau organique donné doit se porter sur celle qui induit le saut résistif le plus élevé.

La mesure de résistivité superficielle des zones traitées exprimée $\Omega/\square$, s'effectue selon la norme CEI 60093.

Sans vouloir être lié par une quelconque théorie scientifique, on peut penser que ce phénomène de saut résistif peut s'expliquer par la diffusion de l'oxygène de l'air vers le réservoir de radicaux libres, puis sa recombinaison très rapide par des mécanismes radicalaires avec les molécules présentes dans cette zone. Ce processus d'oxydation a pour effet de réduire de façon brutale la densité de radicaux libre autrement dit la conductivité superficielle. Cela se traduit lorsque l'on analyse l'évolution temporelle de la résistivité superficielle du matériau organique, par un saut résistif se présentant sous la forme d'une marche. Pour une dose plus forte, ces radicaux libres disparaissent laissant place à du carbone amorphe avec des propriétés électriques très stables dans le temps. Dans ce cas l'évolution temporelle de la résistivité superficielle du matériau organique reste constante. Le procédé selon l'invention est capable d'identifier un saut résistif signalant la présence de ce réservoir de radicaux libres en couche profonde. La hauteur de la marche donne une estimation de la densité de radicaux libres présents dans ce réservoir et sera choisie de manière à être la plus élevée possible.

Outre le renforcement des propriétés hydrophobiques, hydrophiliques, antibactérien intimement liées au greffage de monomères en couche profonde, le procédé permet de durcir en même temps la surface du matériau organique sur une épaisseur inférieure ou égale au micron en créant une couche de carbone amorphe en extrême surface. Cette couche de carbone amorphe peut être obtenue en ajustant la dose d'ions implantés de manière à réticuler totalement le matériau organique en extrême surface et partiellement dans une plus grande profondeur. Les inventeurs ont pu constater que cet effet est particulièrement renforcé pour des ions multichargés multiénergies issus d'une source RCE. Il semble que les ions de charges moins élevées, donc moins énergétiques, participent à la réticulation totale du matériau organique en extrême surface (couche de carbone amorphe) tandis que les ions de charges plus élevées, donc plus énergétiques, participent à la création du réservoir de radicaux libres en couche profondes. Il est ainsi possible créer deux couches successives, l'une en extrême surface totalement réticulée sous la forme de carbone amorphe et l'autre plus profonde greffables ultérieurement avec des monomères. Cette copolymérisation est capable d'apporter avantageusement des couples d'améliorations distincts (dureté/hydrophobie ; dureté/adhérence; dureté/antibactérien etc.).

Le procédé présente l'avantage de créer des barrières hydrofuges ou antibactériennes épaisses, donc efficaces sur la durée ou dans des conditions d'usage sévères sans modifier les propriétés massiques du matériau organique. De fait, on peut envisager de remplacer des flacons en verres par des flacons en plastique devenus, après traitement, imperméables à l'humidité ambiante. Autre exemple, le procédé selon l'invention présente l'avantage d'apporter aux élastomères d'excellentes propriétés de mouillabilité (hydrophile) combinées à une dureté superficielle hautement compatible pour l'application d'un vernis à base aqueuse.

Selon différents modes de réalisation qui peuvent être combinés entre eux :

- la dose d'ions par unité de surface est comprise entre $10^{13}$ ions/cm$^2$ et $5.10^{17}$ ions/cm$^2$ ;
- le matériau polymère appartient à la famille des polymères, des élastomères ou des résines;
- la tension d'accélération des ions est comprise entre 20 kV et 200 kV ;
- les ions sont produits par une source à résonance cyclotronique électronique (RCE) qui a l'avantage d'être compacte et économe en énergie et de produire des ions multichargés multiénergies favorables à la création d'un couche hybride (couche carbone amorphe/ couche greffable).

La présente invention a donc pour objet un procédé selon la revendication 1 de traitement de surface d'un dispositif de distribution de produit fluide, ledit procédé comprenant l'étape de modifier par implantation ionique, au moyen de faisceaux d'ions multichargés et multi-énergies, au moins une surface à traiter d'au moins une partie dudit dispositif en contact avec ledit produit fluide, ladite surface modifiée ayant des propriétés limitant l'apparition et/ou la prolifération de bactéries sur ladite surface modifiée, lesdits ions multichargés étant choisis parmi l'hélium (He), le bore (B), le carbone (C), l'azote (N), l'oxygène (O), le néon (Ne), l'argon (Ar), le krypton (Kr), le xénon (Xe) l'implantation ionique étant réalisée à une profondeur de 0 à 3 $\mu$m.

Ledit procédé comprend un procédé de greffage de monomères en couche profonde dans un matériau organique, comprenant deux étapes successives :

a) une étape de bombardement ionique par un faisceau d'ions pour créer :

- un réservoir de radicaux libres dans une couche (1) d'une épaisseur $e_{rad}$ comprise entre 20 nm à 3000 nm;
- une couche stabilisante (2) s'interposant entre la surface et le réservoir de radicaux libres (1) d'une épaisseur $e_{stab}$ comprise entre 0 nm à 3000 nm;
- les ions du faisceau d'ions sont sélectionnés parmi les ions des éléments de la liste constituée de l'hélium (He), du bore (B), du carbone (C), de l'azote (N), de l'oxygène (O), du néon (Ne), de l'argon (Ar), du krypton (Kr), du xénon (Xe) ;
- la tension d'accélération des ions est supérieure ou égale à 10 kV et inférieure ou égale à 1000 kV ;
- la température de traitement du matériau organique est inférieure ou égale à sa température de fusion;
- on choisit la dose d'ions par unité de surface dans une plage comprise entre $10^{12}$ ions/cm$^2$ et $10^{18}$ ions/cm$^2$

en identifiant par une mesure de l'évolution temporelle de la résistivité superficielle du matériau organique la dose qui induit le saut de marche résistif le plus élevé ;

b) une étape de greffage de monomères consistant à diffuser des monomères (M) au travers d'une couche stabilisante (2) de la surface vers le réservoir de radicaux libres (1) à une température de diffusion $T_d$.

Ces caractéristiques et avantages et d'autres de la présente invention apparaîtront plus clairement dans la description détaillée ci-après, faite notamment en référence aux dessins joints, donnés à titre d'exemples non limitatifs, et sur lesquels

- la figure 1 illustre la formation d'une couche constituée d'une couche de carbone amorphe en extrême surface et d'un réservoir de radicaux libres situé plus en profondeur ;
- la figure 2 illustre l'évolution temporelle caractéristique de la résistivité superficielle d'un matériau organique, brut, traité par un procédé selon l'invention ;
- la figure 3 illustre expérimentalement l'évolution de la résistivité superficielle pour différente doses d'un polycarbonate traité avec des ions He$^+$, He$^{2+}$; la méthode préconisée par le procédé de l'invention permet d'identifier un réservoir de radicaux libres particulièrement favorable au greffage en couche profonde ; cette identification consiste à reconnaître un saut résistif nettement marqué ;
- la figure 4 illustre un premier mode de réalisation d'une surface antibactérienne par le procédé de l'invention ;
- la figure 5 illustre un autre mode de réalisation d'une surface antibactérienne par le procédé de l'invention ; et
- la figure 6 illustre la libération d'ions bactéricides dans un fluide déposé sur une surface antibactérienne traitée selon le procédé de l'invention.

WO2010/100384 décrit un procédé de traitement de surface par implantation ionique par plasmas micro-onde, notamment plasmas à la résonance cyclotron électronique (RCE, e.g d'hélium), notamment sur élastomère, émettant des faisceaux d'ions multichargés, permettant ainsi d'implanter simultanément des ions multi-énergies avec la même tension d'extraction, sur une surface d'un dispositif de distribution de produit fluide: piston de seringue, et ce afin d'en modifier les propriétés mécaniques et chimiques de surface. De même, WO2008/141141 décrit aussi un procédé d'implantation ionique par plasmas micro-onde, notamment plasmas à la résonance cyclotron électronique (RCE, voir [0055], e.g d'hélium, Ar or N, voir Rev.25), notamment sur élastomère ou polymère, émettant des faisceaux d'ions mono et multichargés, permettant ainsi d'implanter simultanément des ions multi-énergies avec la même tension d'extraction, sur une surface d'un dispositif de distribution de produit fluide: dispositif médical pouvant être un réservoir délivrant un médicament (drug-releasing reservoir) ou agent thérapeutique (voir [0002]-[0003]), ou encore valve ou pompe de coeur artificiel ([0017]), et ce afin d'en modifier les propriétés mécaniques et chimiques de surface.)

En particulier, la présente invention prévoit d'utiliser un procédé similaire à celui décrit dans le document WO 2005/085491, qui concerne un procédé d'implantation ionique, et plus particulièrement l'utilisation d'un faisceau d'ions multichargés multi énergies afin de modifier structurellement la surface de matériaux métalliques sur des profondeurs autour du μm pour leur conférer des propriétés physiques particulières. Ce procédé d'implantation a notamment été utilisé pour traiter des pièces réalisées en alliage d'aluminium qui sont utilisées comme moules de fabrication en série de pièces en matière plastique.

De manière surprenante, ce type de procédé s'est avéré adapté pour limiter la formation d'un biofilm sur les surfaces traitées, et ainsi limiter voire empêcher l'apparition et/ou la prolifération de bactéries sur des surfaces destinées à venir en contact avec un produit fluide pharmaceutique dans des dispositifs de distribution du type nasal ou oral. Une telle application de ce procédé d'implantation ionique n'avait jamais été envisagée. La totalité de la description de ce document WO 2005/085491 est donc incorporé dans la présente description à titre de référence.

Les surfaces à traiter peuvent être métalliques, mais également synthétiques, tels que des polymères. Le procédé s'applique aussi à des matériaux tels que le verre ou les élastomères.

De manière simplifiée, le procédé consiste à utiliser une ou plusieurs sources d'ions, telles qu'une source à résonance cyclotronique électronique, dite source RCE. Cette source RCE peut délivrer un faisceau initial d'ions multi-énergies, par exemple ayant un courant total d'environ 10 mA (toutes charges confondues), sous une tension d'extraction pouvant varier de 20 kV à 200 kV. La source RCE émet le faisceau d'ions en direction de moyens de réglage qui assurent la focalisation et le réglage du faisceau initial émis par la source RCE en un faisceau d'implantation d'ions qui vient frapper une pièce à traiter. Selon les applications et les matériaux à traiter, les ions peuvent être choisis parmi l'hélium, le bore, le carbone, l'azote, l'oxygène, le néon, l'argon, le krypton et le xénon. De même, la température maximale de la pièce à traiter varie en fonction de sa nature. La profondeur d'implantation typique est entre 0 et 3 μm, et dépend non seulement de la surface à traiter mais aussi des propriétés qu'on souhaite améliorer.

La spécificité d'une source d'ions RCE réside notamment dans le fait qu'elle délivre des ions mono- et multichargés, ce qui permet d'implanter simultanément des ions multi-énergies avec la même tension d'extraction. Il est ainsi possible d'obtenir simultanément sur toute l'épaisseur traitée un profil d'implantation mieux réparti. Ceci améliore la qualité du traitement de surface.

Avantageusement, le procédé est effectué dans une enceinte mise sous vide grâce à une pompe à vide. Ce vide a pour but notamment d'empêcher l'interception du faisceau par des gaz résiduels et d'éviter la contamination de la surface de la pièce par ces mêmes gaz lors de l'implantation.

Avantageusement, comme notamment décrit dans le document WO 2005/085491, les moyens de réglage ci-dessus mentionnés peuvent comporter, de la source RCE vers la pièce à traiter, les éléments suivants:

- un spectromètre de masse apte à filtrer les ions en fonction de leur charge et de leur masse. Un tel spectromètre est toutefois facultatif si l'on injecte un gaz pur, par exemple un gaz d'azote pur (N2). Il est alors possible de récupérer l'ensemble des ions mono- et multichargés produits par la source pour obtenir un faisceau d'ions multi-énergies.
- une ou plusieurs lentilles pour donner au faisceau d'ions une forme prédéterminée, par exemple cylindrique, avec un rayon prédéterminé.
- un profileur pour analyser lors de la première implantation l'intensité du faisceau dans un plan de coupe perpendiculaire.
- un transformateur d'intensité pour mesurer en continu l'intensité du faisceau d'ions sans l'intercepter. Cet instrument a notamment pour fonction de détecter toute interruption du faisceau d'ions et de permettre l'enregistrement des variations d'intensité du faisceau durant le traitement.
- un obturateur, qui peut par exemple être une cage de Faraday, pour interrompre la trajectoire des ions à certains moments, par exemple lors d'un déplacement sans traitement de la pièce.

Selon une forme avantageuse de réalisation, la pièce à traiter est mobile par rapport à la source RCE. La pièce peut par exemple être montée sur un support mobile dont le déplacement peut être commandé par une machine à commande numérique. Le déplacement de la pièce à traiter est calculé en fonction du rayon du faisceau, des contours externes et internes des zones à traiter, de la vitesse de déplacement constante ou variable en fonction de l'angle du faisceau par rapport à la surface et du nombre de passes précédemment réalisées.

Une mise en oeuvre possible du procédé de traitement est la suivante. On fixe la pièce à traiter sur un support approprié dans une enceinte, puis on ferme l'enceinte et on y instaure un vide poussé au moyen d'une pompe à vide. Dès que les conditions de vide sont atteintes, on procède à la production et au réglage du faisceau d'ions. Lorsque ledit faisceau est réglé, on lève l'obturateur et on actionne la machine à commande numérique qui exécute alors le déplacement en position et en vitesse de la pièce à traiter devant le faisceau en une ou plusieurs passes. Lorsque le nombre de passes requis est atteint, on baisse l'obturateur pour couper le faisceau, on arrête la production du faisceau, on casse le vide en ouvrant l'enceinte à l'air ambiant, on arrête éventuellement le circuit de refroidissement et on sort la pièce traitée hors de l'enceinte.

Pour diminuer la température liée au passage du faisceau d'ions en un point donné de la pièce à traiter, on peut soit augmenter le rayon du faisceau (donc réduire la puissance par cm$^2$), soit augmenter la vitesse de déplacement. Si la pièce est trop petite pour évacuer par rayonnement la chaleur liée au traitement, on peut soit diminuer la puissance du faisceau (donc augmenter la durée de traitement), soit mettre en marche le circuit de refroidissement.

Le procédé de l'invention est non polluant, notamment du fait qu'il ne nécessite pas de produits chimiques. Il est réalisé à sec, ce qui évite les périodes de séchages relativement longues des procédés de traitement liquides. Il ne nécessite pas une atmosphère stérile en dehors de l'enceinte à vide, et il peut donc être réalisé en tout endroit souhaité. Un avantage particulier de ce procédé est qu'il peut être intégré dans la chaine de montage du dispositif de distribution de produit fluide, et opérer en continu dans cette chaine. Cette intégration du procédé de traitement à l'outil de production simplifie et accélère le processus de fabrication et d'assemblage dans son ensemble, et impacte donc positivement son coût.

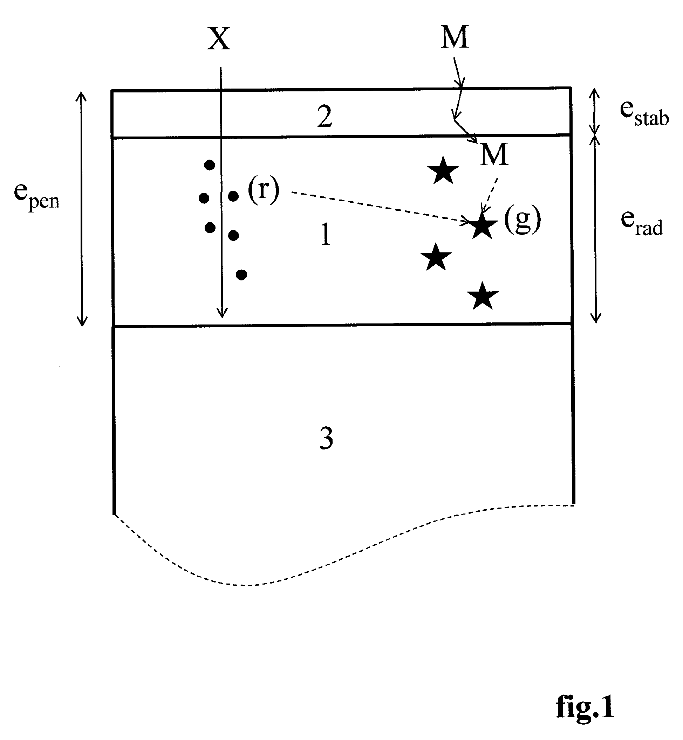

Les figures 1 à 6 illustrent des modes de réalisation avantageux.

Selon des exemples de mise en oeuvre du procédé des échantillons de polycarbonate ont fait l'objet d'études pour un traitement avec des ions hélium émis par une source RCE.

Le faisceau d'ions d'une intensité de 5 mA comprend des ions He$^+$, He$^{2+}$ avec comme répartition (He$^+$/He$^{2+}$) = 10; la tension d'extraction et d'accélération est de 35 kV ; l'énergie de He$^+$ est de 35 keV et celle de He$^{2+}$ de 90 keV.

L'échantillon à traiter se déplace par rapport au faisceau avec une vitesse de déplacement à 40 mm/s avec un pas d'avancement latéral à chaque retour de 1 mm. Pour atteindre la dose nécessaire le traitement se fait en plusieurs passes.

L'évolution temporelle de la résistivité de la surface du polycarbonate a été effectuée en s'appuyant sur la norme CEI 60093 qui préconise la mesure au bout d'une minute de la résistance électrique existant entre deux électrodes, l'une constituée d'un disque de diamètre d, l'autre d'une couronne centrée sur le disque et d'un rayon interne D. Ces électrodes sont posées sur la surface du polycarbonate et sont soumises à une tension de 100 V. D est égal à 15 mm et d est égal à 6 mm. La mesure de résistivité n'est possible que pour des valeurs inférieures à 10$^{15}$ Ω/□.

Selon un premier exemple de mise en oeuvre de la présente invention, des échantillons de PP (polypropylène) ont fait l'objet d'étude de greffage avec de l'acide acrylique pour un traitement avec des ions hélium émis par une source RCE.

Le faisceau d'ions d'une intensité de 300 μA comprend des ions He$^+$, He$^{2+}$ avec comme répartition (He$^+$/He$^{2+}$) = 10; la tension d'extraction et d'accélération est de 35 kV ; l'énergie de He$^+$ est de 35 keV et celle de He$^{2+}$ de 90 keV. L'échantillon à traiter se déplace par rapport au faisceau avec une vitesse de déplacement à 80 mm/s avec un pas d'avancement latéral à chaque retour d'environ 3 mm. Pour atteindre la dose nécessaire le traitement se fait en plusieurs passes.

Les échantillons en polyéthylène PP ont été bombardés selon différentes doses correspondant à 2.10$^{14}$, 5.10$^{14}$, 10$^{15}$ ions/cm$^2$.

Une seule condition de greffage a été mise en oeuvre: immersion pendant 24 h dans une solution d'acide acrylique (CH2=CO-OH) dosée à 10% en poids, maintenue à 40°C

Des mesures d'angles de contact de gouttes ont permis de valider la modification de la mouillabilité de la surface après greffage, caractérisé par le passage d'un comportement hydrophobe vers un comportement hydrophile.

Ces résultats sont rassemblés dans le tableau 1.

Tableau 1

| Echantillon | Angle de contact(°) |
|---|---|
| Brut | 76° |
| Traité 2.10$^{14}$ ions/cm$^2$ + immersion | 74° |
| Traité 5.10$^{14}$ ions/cm$^2$ + immersion | 64° |
| Traités 10$^{15}$ ions/cm$^2$ + immersion | 66° |

Le comportement du PP change, l'échantillon brut présente un comportement plutôt hydrophobe (angle de contact de 76°) tandis que les échantillons traités présente un comportement qui tend plutôt à être l'hydrophile (angle de contact plus petit de 64°). On observe que le comportement hydrophile est nettement amélioré pour des doses comprises entre 5.10$^{14}$ et 10$^{15}$ ions/cm$^2$. On constate que l'analyse FTIR du PP traité avec He indique une dose du même ordre de grandeur que celles obtenues par mesure de conductivité superficielle sur du PC traité avec He.

Selon un deuxième exemple de mise en oeuvre du procédé des échantillons de polypropylène ont fait l'objet d'études de greffage avec de l'acide acrylique pour un traitement avec des ions azote émis par une source RCE.

Le faisceau d'ions d'une intensité de 300 μA comprend des ions N+$^+$, N$^{2+}$, N$^{3+}$ avec comme répartitions respectives 60%, 40%, 10%; la tension d'extraction et d'accélération est de 35 kV ; l'énergie de N$^+$ est de 35 keV, celle de N$^{2+}$ de 90 keV, et celle N$^{3+}$ de 105 keV.

Les échantillons en PP ont été bombardés selon différentes doses à 2.10$^{14}$, 5.10$^{14}$, 10$^{15}$, 5.10$^{15}$ ions/cm$^2$.

L'échantillon à traiter se déplace par rapport au faisceau avec une vitesse de déplacement à 80 mm/s avec un pas d'avancement latéral à chaque retour de 3 mm. Pour atteindre la dose nécessaire

Deux conditions de greffage ont été mises en oeuvre dont les résultats sont rassemblés dans le tableau 2 :

• Une solution d'acide acrylique (CH2=CH-COOH) de 10% en volume, maintenue à 40°C.
• Une solution d'acide acrylique (CH2=CH-COOH) de 10% en volume, maintenue à 60°C.

Tableau 2

| Echantillon | Angle de contact(°) | |
|---|---|---|
| | Greffage à 40°C | Greffage à 60°C |
| Brut | 82° | 82° |
| Brut immergé | 81° | 80° |
| Traité 2.10$^{14}$ ions/cm$^2$ et immergé | 58° | 60° |
| Traité 5.10$^{14}$ ions/cm$^2$ et immergé | 70° | 68° |
| Traités 10$^{15}$ ions/cm$^2$ et immergé | 75° | 70° |

(suite)

| Echantillon | Angle de contact(°) | |
|---|---|---|
| | Greffage à 40°C | Greffage à 60°C |
| Traités $5.10^{15}$ ions/cm$^2$ et immergé | 65° | 75° |

On observe que le greffage a bien eu lieu pour tous les échantillons traités et immergés dans une solution d'acide acrylique à 40° ou 60°. Des échantillons bruts et immergés dans la solution de greffage n'ont révélés aucun changement en terme de mouillabilité ce qui révèle que le bombardement ionique dans les conditions préconisées par le procédé de l'invention est bien à l'origine du greffage. Pour des doses inférieures à $10^{15}$ ions/cm$^2$, les angles de contacts semblent relativement comparables à 2° près. Pour les doses de $10^{15}$, $5.10^{15}$ ions/cm$^2$, on note une inversion de l'effet : pour une dose de $10^{15}$ ions/cm$^2$ et une immersion à 60° l'angle de contact de la goutte d'eau est plus faible que pour l'immersion à 40° (70°<75°) ; pour une dose de $5.10^{15}$ ions/cm$^2$ et une immersion à 60° l'angle de contact de la goutte d'eau est plus forte que pour l'immersion à 40° (65°<75°). Sans vouloir être lié par une quelconque théorie scientifique, on peut penser que la couche stabilisante est de faible épaisseur aux faibles doses ($2.10^{14}$, $5.10^{14}$ ions/cm$^2$). Cette couche est parcourue par les molécules d'acide acrylique en un temps relativement cours aussi bien à 40°C ou 60°C, avant que puisse avoir lieu un début de recombinaison des radicaux libres entre eux, au coeur même du réservoir créée par le bombardement ionique. Le greffage de l'acide acrylique avec les radicaux libre du réservoir est dans ce cas total. L'angle de contact a tendance à augmenter en même temps que l'épaisseur de la couche stabilisante qui sépare le réservoir de radicaux libres de la surface. Lorsqu'on augmente la dose, autrement dit lorsque l'épaisseur de la couche stabilisante atteint un certain seuil, la température joue plutôt en faveur d'une recombinaison des radicaux libres entre eux au détriment du greffage. L'acide acrylique n'a alors plus le temps d'atteindre le réservoir de radicaux libres pour y être greffé. De fait l'angle de contact de la goutte à 60°C est plus fort qu'à 40°C. Les inventeurs ont pu conclure qu'il est préférable dans ce cas greffer à 40°C voire à température ambiante plutôt qu'à 60°C.

Le greffage de l'acide acrylique a également été confirmé par un moyen d'investigation plus fin, l'analyse FTIR (Fast Transform Infra Red). On peut observer dans le spectre IR des échantillons et pour différentes doses, pic d'absorption autour de 1710 cm-1 (pic d'absorption du groupement carbonyle : C=O) ; l'apparition pic d'absorption autour de 3200 cm-1 (pic d'absorption du groupement hydroxyle (OH)). Ces deux groupes fonctionnels carbonyle et hydroxyle sont absents du PP et ne peuvent donc provenir que l'acide acrylique.

On donne dans les tableaux 3 et 4 ci-dessous les résultats selon les températures d'immersion respectives de 40°C et 60°C :

Tableau 3

| Dose + Immersion acide acrylique 40°C | Transmittance à 1710 cm-1 | Transmittance à 3200 cm-1 |
|---|---|---|
| Brut | 97,5% | 97,5% |
| $10^{14}$ | 95% | 96 |
| $5.10^{14}$ | 86% | 92% |
| $10^{15}$ | 87% | 88% |
| $5.10^{15}$ | 89% | 92% |

On constate que la dose optimale pour laquelle le pic d'absorption (diminution de transmittance) est le plus important est située autour de $5.10^{14}$ ions/cm$^2$.

Tableau 4

| Dose+ Immersion acide acrylique 60°C | Transmittance à 1710 cm-1 | Transmittance à 3200 cm-1 |
|---|---|---|
| Brut | 97,5% | 97,5% |
| $10^{14}$ | 95% | 92% |
| $5.10^{14}$ | 89% | 89,5% |
| $10^{15}$ | 88,5% | 88,5% |
| $5.10^{15}$ | 90% | 89% |

On constate que pour une immersion à 60°C, la dose optimale pour laquelle le pic d'absorption (diminution de transmittance) est le plus important est située autour de $10^{15}$ ions/cm$^2$. Ceci est vrai, aussi bien pour les groupements CO (1710 cm-1) que pour les groupements OH (3200 cm-1). Les pics d'absorption sont plus faibles à 60°C qu'à 40°C confirmant ainsi qu'une partie des radicaux libres se recombinent en partie entre eux sous l'effet de la température.

Les inventeurs ont pu constater sur la base d'essais préliminaires et d'extrapolation qu'il était possible de pour tout type d'ion à une énergie donnée, de calculer la dose correspondant au saut de marche résistif le plus élevé, en s'appuyant sur les résultats obtenus dans les mêmes conditions pour un autre type d'ions avec une autre énergie. La relation est la suivante :

$$N1 \times Eion(E1) = N2 \times Eion(E2)$$

où :

- N1 est la dose (le nombre d'ions par unité de surface) associé au saut de marche résistif le plus élevé d'un ion (1).
- E1 l'énergie de l'ion (1).
- $E_{ion}(E1)$ est l'énergie d'ionisation de l'ion (1) en début de parcours dans le polymère. Cette énergie correspond à l'énergie cédée par l'ion (1) aux électrons du polymère sous forme d'ionisation.
- N2 est la dose (le nombre d'ions par unité de surface) associé au saut de marche résistif le plus élevé d'un ion (2).
- E2 l'énergie de l'ion (2).
- $E_{ion}(E2)$ est l'énergie d'ionisation de l'ion (2) en début de parcours dans le polymère.

Cette énergie d'ionisation est fonction de la nature et de l'énergie et de la nature du polymère. Des méthodes et données permettant de faire ces calculs sont notamment divulguées dans les publications « The Stopping and Range of Ions in Matter » by J.F. Ziegler, volumes 2-6, Pergamon Press, 1977-1985, « The Stopping and Range of Ions in Solids » by J.F. Ziegler, J.P. Biersack and U. Littmark, Pergamon Press, New York, 1985 et J.P. Biersack and L. Haggmark, Nucl. Instr. and Meth., vol. 174, 257, 1980.

En outre, des logiciels ont été développés et commercialisés afin de faciliter ou d'effectuer de tels calculs, comme par exemple les logiciels commercialisés sous les noms « SRIM » (« *The Stopping and Range* of *Ions in Matter* ») et « TRIM » (« *The Transport of Ions in Matter* »), développés notamment par James F. Ziegler.

A titre d'exemple, on obtient pour le PP (polypropylène) le tableau 5 de correspondance suivant :

Tableau 5

| Type d'ion | Energie(Kev) | Eion(ev/Angstroem) | N(ion/cm$^2$) |
|---|---|---|---|
| He | 35 | 10 | $10^{15}$ |
| N | 50 | 20 | $5.10^{14}$ |
| Ar | 40 | 20 | $5.10^{14}$ |

La première ligne du tableau contient reprend des données expérimentales connues: He type d'ion mis en oeuvre, 35 keV énergie de l'ion utilisé, 10 eV/Angstroem énergie d'ionisation cédée par l'hélium en début de parcours dans le PP (fournie par TRIM&SRIM). La dose $10^{15}$ ion/cm$^2$ est la dose identifiée par l'expérience correspondant au saut de marche résistif pour le PC, sachant que le PC présente une énergie d'ionisation de (9,5 eV/Angstroem) quasiment identique à celle du PP.

Dans la deuxième ligne on connaît la nature de l'ion N, son énergie 50 keV, son énergie d'ionisation estimée par TRIM&SRIM à 20 eV/Angstroem. On déduit la dose correspondant au saut de marche résistif le plus élevé en appliquant la relation N = (10 x $10^{15}$)/20 = $5.10^{14}$ ions/cm$^2$. Cette dose est relativement proche de celle déduite par FTIR pour le PP (cf. tableau 3)

La troisième ligne constitue un autre exemple de greffage avec l'argon à valider. Pour celui-ci on déduit une dose correspondant au saut de marche résistif le plus élevé qui se situerait autour de $5.10^{14}$ ion/cm$^2$ autrement dit relativement proche de celle obtenue avec le faisceau d'azote.

Le procédé de l'invention se caractérise par les avantages suivant :

- Création d'un réservoir de radicaux libre d'une capacité optimale accessible aux monomères de la solution. Les autres techniques de greffage par plasmas, par faisceau électroniques et rayon gamma ont pour inconvénient de créer des radicaux libres en profondeur, inaccessibles aux monomères, et qui dégradent les propriétés de masse du matériau.

- Conservation de ce réservoir de radicaux libres à température ambiante et sur une longue durée sur une longue durée des radicaux libres en condition ambiante avant greffage. On peut greffer le polymère traité plusieurs jours après le bombardement. Ce n'est pas le cas pour les autres techniques de greffage par plasmas, par faisceau électroniques et rayon gamma. Ces techniques ne créent pas de couche stabilisante, les polymères ainsi traités doivent être maintenus dans l'obscurité à une basse température, inférieure à - 20°C, avant greffage.
- Saturation de la couche greffée en monomère.

Il existe deux modes réalisation pour créer une couche greffée stockant et libérant des ions connus pour leur action bactéricide comme par exemple les ions argent ($Ag^+$), les ions cuivre ($Cu^{2+}$) ou les ions zinc ($Zn^{2+}$). Le choix du mode de réalisation dépendra essentiellement du cout de mise en oeuvre : on peut citer par exemple le cout des monomères à greffer, le nombre d'opération à effectuer pour obtenir l'effet antibactérien (immersion dans une ou deux solutions).

Le premier mode de réalisation consiste à bombarder le polymère avec des ions puis à l'immerger dans une solution de sels métalliques comme par exemple dans une solution d'acrylate métallique ($CH2=CH-COO-+(M^+)$) ou encore ($2\,CH2=CH-COO-+(M^{2+})$). On peut prendre par exemple l'acrylate de cuivre, l'acrylate d'argent ou encore d'acrylate de zinc. L'acrylate de cuivre connu pour ses propriétés biocides, il est notamment utilisé dans des peintures dites « antifouling » pour coques de bateau. Le but est d'éviter l'accrochage d'organisme marin. Dans la législation maritime le relargage de cuivre ne doit pas, pour des raisons d'impact sur l'environnement, excéder plus 20 microgramme par jour et par $cm^2$ dans les 14 premiers jours de la mise en contact avec l'eau de mer. Le principe du greffage antibactérien est le suivant : l'acrylate réagit avec les radicaux libres pour se fixer au substrat entrainant avec lui l'ion métallique bactéricide fixé faiblement sur sa terminaison $CO^{2-}$. L'ion métallique peut ensuite être libéré vers l'extérieur pour exercer son action bactéricide.

Le deuxième mode de réalisation comprend de son coté deux étapes :

- Une première étape ou l'on bombarde et ou l'on greffe le polymère avec des monomères ayant pour caractéristiques d'établir des liaisons faibles (de type chélation) avec des ions métalliques. On citera comme exemple, l'acide acrylique : les doublets électroniques non liants du groupe hydroxyle de l'acide acrylique sont en effet capables de piéger par chélation des ions métalliques.
- Une deuxième étape ou l'on charge la couche greffée avec ions métalliques bactéricides en l'immergeant dans une solution contenant ces mêmes ions. Une fois stockés dans la couche greffée, ces ions métalliques bactéricides sont libérés dès qu'ils entrent en contact avec un fluide déposé sur la couche greffée. Les ions métalliques bactéricides diffusent dans le fluide et exercent leur action bactéricide à condition que leur concentration dépasse un seuil de concentration bactéricide spécifique à la nature de l'ion. Pour l'ion argent ($Ag^+$), on sait qu'un seuil de concentration à 20 ppm (parties pour million), autrement dit à 20 mg par kg est hautement bactéricide pour détruire des bactéries comme Staphylococcus aureus (Staphylococcus aureus résistant à la méthicilline ou MRSA) Enterococcus faecium (Enterococcus résistant à la vancomycine ou VRE) Enterococcus fecalis Burkholderia cepacia Alcaligenes sp. Pseudomonas eruginosaklebsiella pneumoniae Pseudomonas sp. Acinetobacter sp. itrobacter koseri. Pour 1 cm3 de fluide déposé sur un $cm^2$ d'une couche greffée avec de l'acide acrylique et chargée avec des ions $Ag^+$, il faut libérer l'équivalent de 20 $\mu g/cm^2$ d'ions $Ag^+$ pour avoir une effet bactéricide efficace. Pour le cuivre le seuil de concentration bactéricide est d'environ 10 ppm autrement dit de 10 $\mu g/cm3$. Il existe de nombreuses solutions pour charger la couche greffée avec des ions bactéricides. On citera par exemple les solutions de sulfate de cuivre ($CuSO4$), de nitrate d'argent ($AgNO3$) ou encore de chlorure d'argent.

Dans les deux modes de réalisations, la couche greffée antibactérienne agit, comme, un échangeur d'ions bactéricides dont on peut avantageusement régler les caractéristiques pour:

- Garantir un effet antibactérien efficace sur la durée:

  o Dépassement du seuil de concentration bactéricide,
  o Maintien de cet effet sur une durée significative au regard de l'application visée.

- Limité l'impact du traitement sur l'environnement ou sur la santé.
- Réduire les couts de mise en oeuvre, par exemple en diminuant la quantité de métal précieux d'ou proviennent ions $Ag^+$.

Les inventeurs ont développé un modèle de greffage et de stockage d'ions métalliques permettant d'établir une formule utile pour effectuer des prédictions sur la capacité de stockage en ions métalliques en fonction des paramètres du bombardement.

Ce modèle s'appuie, d'une part sur la nature spécifique de la couche greffée, telle qu'elle a pu être observée expérimentalement (réservoir de radicaux libres affleurant la surface et protégé par une couche stabilisante de carbone amorphe), d'autre part sur des considérations d'encombrement stérique dont l'effet est de limiter le greffage indépendamment du nombre de radicaux libres en présence.

Ce modèle intègre les points suivants:

- Pour une dose ou se produit le saut de marche résistif le plus élevé:

  o Les monomères constituant le polymère présentent de façon équiprobable un nombre de radicaux libres qui vont en décroissant de l'extrême surface à la fin de parcours de l'ion.
  o Ces radicaux libres sont préservés par la couche stabilisante. Leur nombre est constant jusqu'au moment du greffage.

- Le greffage des monomères à greffer est limité par la taille des monomères constituant le polymère. Lorsque les monomères à greffer sont de taille comparable aux monomères constituant le polymère, il n'est pas possible de greffer plus d'un monomère sur un monomère constituant le polymère. La règle de greffage est la suivante : le nombre de monomères Ng que l'on peut greffer sur un monomère constituant le polymère est compris entre à (Lp/Lg) et (Lp/Lg)-1 si Lp > Lg, ou Lg est la longueur du monomère à greffer et Lp la longueur du monomère constituant le polymère ; si Lp < Lg, $N_G$ est égale à Lp/(Lg+1).
- Les monomères greffés établissent des liens faibles avec les ions métalliques bactéricides. On peut déduire le nombre d'ions bactéricides liés à un monomère greffé en considérant sa composition chimique. Par exemple un monomère greffé comme l'acide acrylique ne peut accueillir par chélation, qu'un seul ion $Ag^+$ ou $Cu^{2+}$ qui se fixe sur un des deux doublets non liants du groupement hydroxyle (OH).

A partir de ces hypothèses les inventeurs ont pu établir la formule suivante :

$$N_{ion} = (1/2).6,02x10^{23}.E_p.(\rho/M_{mol}).K.A$$

où :

- $N_{ion}$ représente le nombre d'ions bactéricides stockés et libérables par unité de surface.
- 1/2 : représente un facteur correctif qui prend en compte la décroissance linéaire de radicaux libres de l'extrême surface à la fin du parcours de l'ion.
- $E_p$ : représente l'épaisseur bombardée et greffée. Cette épaisseur est fonction de l'énergie de l'ion, de sa nature et de la nature du polymère. Elle est calculable avec le logiciel TRIM&SRIM.
- p : représente la densité volumique du polymère.
- $M_{mol}$ : représente le poids molaire du monomère constituant le polymère
- K : représente le nombre moyen de monomères greffés par monomère constituant le polymère.

  o Si Lp > Lg, K est compris entre Lp/Lg et Lp/Lg-1, on prendra la valeur moyenne : K = (2x(Lp/Lg)-1)/2.
  o Si Lp < Lg, K = Lp/(Lg+1).
  o Ce nombre K peut être affiné, corrigé voire déduit directement par l'expérience. On utilise pour cela une technique appelée RBS (Rutherford Back Scattering) qui permet de pulvériser couche par couche une surface pour déduire par spectrométrie de masse la composition des éléments pulvérisés. Il est possible, par exemple, d'évaluer le nombre d'atome d'oxygène par unité de surface attribuable à la présence d'acide acrylique greffé et de déduire le nombre de monomères d'acide acrylique par unité de surface sachant d'une part qu'il faut deux atome d'oxygène par monomère d'acide acrylique et que d'autres part ces atomes ne peuvent pas provenir du polymère. On peut ainsi corriger K en lui appliquant un facteur correctif expérimental.

- A : le nombre d'ions métalliques liés par monomère greffés (stockables et libérables). On peut déduire A à partir de la composition chimique du monomère greffé. Par exemple pour un monomère d'acrylate d'argent, il n'y a qu'un seul ion argent (Ag+) par monomère d'acrylate greffé : A=1. Autre exemple, pour un monomère d'acide acrylique, on peut fixer par chélation qu'un seul ion argent ($Ag^+$) sur le seul groupement hydroxyle (OH) : A =1. On considère dans ce modèle que tous les ions métalliques stockables sont totalement libérables. Ce nombre A peut être corrigé voire déduit par des mesures extrêmement sensibles, de l'ordre du µg (microgramme), effectuées avec une microbalance. Pour cela il faut évaluer la différence en poids exprimés en $\mu g/cm^2$ d'un polymère greffé, avant et après immersion dans une solution d'ions métalliques bactéricides, ou encore d'une polymère greffé chargé d'ions métalliques bactéricides, avant et après immersion dans de l'eau désionisée On peut ainsi corriger A en lui appliquant un facteur correctif expérimental.

On peut déduire la charge surfacique Cs, définie comme la masse d'ions métalliques bactéricides stockés et libérables par unité de surface:

$$Cs = (N_{ion}/N_{at})x\ \rho$$

où

- $N_{ion}$ représente le nombre d'ions métalliques bactéricides stockés et libérables par unité de surface.
- $\rho$ représente la densité volumique du métal dont sont fait les ions métalliques bactéricides
- $N_{at}$ représente le nombre d'atome par unité de volume du métal dont sont faits les ions métalliques bactéricides

Par cette formule il est possible d'obtenir des estimations du nombre d'ions stockés et libérables dans une couche greffée de polymère pour évaluer et prédire l'effet bactéricide sur un volume de fluide donné.

On considère par exemple le cas d'un PP (polypropylène) bombardé selon trois types d'ions He, N, Ar de même énergie, avec différentes doses calculés pour obtenir une couche stabilisante et un réservoir de radicaux libre optimal (saut de marche résistif le plus élevé), que l'on greffe ensuite avec de l'acide acrylique, que l'on immerge enfin dans une solution d'argent ou de cuivre. On initialise les paramètres de la formule avec les valeurs suivante:

- densité volumique du polymère PP : $\rho$ = 0,9 g/cm3
- poids molaire du monomère du polymère : $M_{mol}$ = 42 g (monomère :

        CH2=CH-CH3)

- déduction du nombre de monomères greffés par monomère constituant le polymère : K = 0,5 ; La taille du monomère à greffer (CH2=CH-COOH) est sensiblement comparable à celle du monomère du polymère (CH2=CH-CH3).
- Déduction du nombre d'ion métallique $Ag^+$ ou $Cu^{2+}$ liés par monomère greffé : A= 1

Le calcul de la charge surfacique d'ions métalliques bactéricides stockés et libérés, avec la connaissance des seuils de concentration bactéricide, permet de prédire le volume de fluide pouvant faire l'objet d'une action bactéricide efficace.

Par exemple, pour des paramètres de bombardement et de greffage fixés, suivi d'une immersion dans une solution d'ions $Ag^+$, on obtient les estimations de charges surfaciques reportées dans le tableau 6:

Tableau 6

| Type d'ion | Energie (keV) | Epaisseur ($\mu$m) | $N_{ion}$ libérables $Ag^+$ (ions/cm$^2$) | Charge surfacique Ag+ ($\mu$g/cm$^2$) |
|---|---|---|---|---|
| He | 35 | 0,7 | $2,1x10^{17}$ | 37,8 |
| N | 35 | 0,25 | $7,25x10^{16}$ | 12,95 |
| Ar | 35 | 0,12 | $3,6x10^{16}$ | 6,48 |

On constate que la charge surfacique d'ions $Ag^+$ stockés et libérables par une couche bombardée avec He, greffée avec de l'acide acrylique puis immergée dans une solution d'ions $Ag^+$, présente des caractéristiques hautement bactéricides pour traiter un volume de fluide d'environ 1,9 cm$^3$ (le seuil de concentration bactéricide de Ag+ est de 20 ppm autrement dit à 20 $\mu$g/cm$^3$). Pour un bombardement avec N, la charge surfacique d'ions $Ag^+$ stockés et libérables est moindre mais reste toujours aussi efficace pour traiter 0,65 cm$^3$. Pour un bombardement à l'Ar, la charge surfacique d'ions $Ag^+$ stockés et libérables permet de traiter efficacement un film de fluide de 2 mm d'épaisseur. On peut ainsi moduler les propriétés bactéricides d'une surface traitée selon le procédé de l'invention en fonction des applications visées qu'il s'agisse d'une goutte de fluide, d'un film de fluide etc.

Pour des paramètres de bombardement et de greffage fixés, suivi d'une immersion dans une solution d'ions $Cu^{2+}$, on obtient les estimations de charges surfaciques reportées dans le tableau 7:

Tableau 7

| Type d'ion | Energie (keV) | Epaisseur ($\mu$m) | $N_{ion}$ libérable $Cu^{2+}$ (ions/cm$^2$) | Charge surfacique $Cu^{2+}$ ($\mu$g/cm$^2$) |
|---|---|---|---|---|
| He | 35 | 0,7 | $2,1 \times 10^{17}$ | 21 |
| N | 35 | 0,25 | $7,25 \times 10^{16}$ | 7,25 |
| Ar | 35 | 0,12 | $3,6 \times 10^{16}$ | 3,6 |

Comme pour les ions Ag+, on constate que la charge d'ions $Cu2^+$ stockés et libérables par une couche bombardée avec He, greffée avec de l'acide acrylique puis immergée dans une solution d'ions $Cu2^+$, présente des caractéristiques hautement bactéricides pour traiter un volume de fluide d'environ 2,1 cm$^3$ (le seuil de concentration bactéricide de $Cu^{2+}$ est de 10 ppm autrement dit égal à 10 $\mu$g/cm$^3$).

Une autre approche consiste pour un type d'ion donné, à ajuster l'énergie de l'ion, autrement dit adapter la profondeur de l'épaisseur de traitement Ep, pour stocker et libérer une charge d'ions métalliques bactéricides suffisante pour dépasser le seuil de concentration bactéricide (spécifique aux ions métalliques bactéricides) dans un fluide de volume et de surface de contact donnés. La charge d'ions métalliques libérables dans le fluide est proportionnelle à la surface de contact du fluide avec la surface bactéricide.

Par exemple, pour du PP bombardé avec un seul type d'ion azote avec 3 énergies possibles 35 keV, 50 keV, 70 keV et greffé avec de l'acide acrylique, immergé ensuite dans une solution de cuivre $Cu^{2+}$, on peut estimer les charge surfaciques respectives d'ions $Cu^{2+}$ stockés et libérables, dans le but d'identifier l'énergie qui permet de dépasser un seuil de concentration bactéricide égal à (10 $\mu$g/cm$^3$) dans un volume de fluide de 1 cm3 dont la surface de contact est de 1 cm$^2$. Les estimations de charge surfacique sont reportées dans le tableau 8:

Tableau 8

| Type d'ion | Energie (keV) | Epaisseur ($\mu$m) | $N_{ion}$ libérable $Cu^{2+}$ (ions/cm$^2$) | Charge $Cu^{2+}$ ($\mu$g/Cm$^2$) |
|---|---|---|---|---|
| N | 35 | 0,25 | $7,25 \times 10^{16}$ | 7,25 |
| N | 50 | 0,3 | $8,7 \times 10^{16}$ | 8,7 |
| N | 70 | 0,4 | $11,6 \times 10^{16}$ | 11,6 |

On déduit de ce tableau qu'il faut une énergie d'environs 60 keV pour créer une charge bactéricide suffisante de 10 $\mu$g/cm$^2$ pour traiter 1 cm3 s'étalant sur 1 cm$^2$.

Le procédé permet de déterminer les paramètres de bombardement ionique pour créer une couche greffée qui présente des caractéristiques optimales (hydrophile, hydrophobe, antibactérien, échangeur d'ions métalliques), en laissant en suspens les multiples conditions de mise en oeuvre : nature, température et concentration des solutions de monomères à greffer, d'ions métalliques à charger dans la couche greffée. Ces conditions de mise en oeuvre agissent uniquement sur la cinétique chimique (rapidité pour obtenir le résultat). Ces conditions ont peu d'effet effet voire pas du tout sur le résultat lui-même. Ces conditions de mise en oeuvre sont laissées à l'appréciation de l'industriel qui doit les régler lors d'essais préliminaires pour répondre à une cadence de production, à des couts économiques, etc. En règle générale, les inventeurs préconisent des essais préliminaires avec des solutions qui n'excèdent pas 40°C pour éviter les recombinaisons des radicaux libres avant greffage et des concentrations inférieures à 10% en volume pour permettre une bonne homogénéité de la solution durant le greffage ou durant le chargement en ion bactéricide.

Les spectres d'action des ions Ag$^+$ et $Cu^{2+}$ sur les agents bactériens ou fongiques se recouvrent partiellement, les premiers étant plus efficaces, voire pas du tout par rapport aux seconds pour traiter une bactérie ou un champignon. Selon plusieurs modes de réalisation d'une surface bactéricide, on peut élargir les spectres d'action des ces ions en immergeant par exemple un PP bombardé et greffé avec de l'acide acrylique, dans des solution d'ions métalliques bactéricides $Cu^{2+}$ et/ou Ag$^+$ simultanément ou séquentiellement dans un sens ou dans l'autre, dans le but d'obtenir in fine des proportions d'ions métalliques bactéricides stockés spécifiques, par exemple un stockage en ions métalliques bactéricides constitués de 70% d'ions argent (Ag$^+$) et 30% d'ions cuivre ($Cu^{2+}$).

La figure 1 représente la structure d'une épaisseur de matériau organique produite par le bombardement ionique selon le procédé de l'invention. Lorsqu'un ion (X) pénètre le matériau organique dans une épaisseur $e_{pen}$, il produit sur son passage des radicaux libres. Au delà dans la couche (3), le matériau organique conserve ses propriétés d'origine. Les radicaux libres en extrême surface se recombinent très rapidement entre eux dans une zone (2) pour créer préférentiellement et par réticulation une couche stable constituée essentiellement de carbone amorphe dans une épaisseur $e_{stab}$. Les radicaux libres situés plus en profondeur constituent une couche plus réactive (1) d'épaisseur $e_{rad}$,

propice au greffage (1) On qualifie cette couche (1) de réservoir de radicaux libre (r). Ces radicaux libres sont disponibles pour participer au greffage ultérieur de monomères (M). Il faut noter que, la zone (2) peut ne pas exister si la dose d'énergie cédée par les ions incidents en extrême surface n'est pas suffisante pour provoquer une réticulation totale en extrême surface. Dans ce cas la couche stable (2) n'existe pas; La couche de matériau organique accessible aux ions incidents (X) dans une épaisseur se confond avec le réservoir de radicaux libre (1), autrement dit $e_{pen}$ est égale à $e_{rad}$. Dans ce cas le réservoir de radicaux libres (1) est en contact direct avec l'extérieur. Le greffage effectué dans une seconde étape, consiste à faire diffuser un monomère (M) de la surface du matériau organique vers le réservoir de radicaux libres (1) au travers d'une couche stabilisée de carbone amorphe(2) qui peut comme on vient de le voir ne pas exister. Après diffusion dans le réservoir de radicaux libre (1), le monomère (M) réagit avec (r) pour donner un composé chimique de greffage (g) présentant les propriétés hydrophile, hydrofuge ou antibactérienne du monomère d'origine. L'épaisseur $e_{rad}$ est comprise entre 20 nm et 3000 nm correspondant au parcours minimal et maximal des ions incidents compte tenu de leur énergie. L'épaisseur $e_{stab}$ varie selon que l'épaisseur traitée est totalement, peu ou pas réticulée sous forme de carbone amorphe entre 3000 nm et 0 nm. La règle veut que $e_{pen} = e_{rad} + e_{stab}$.

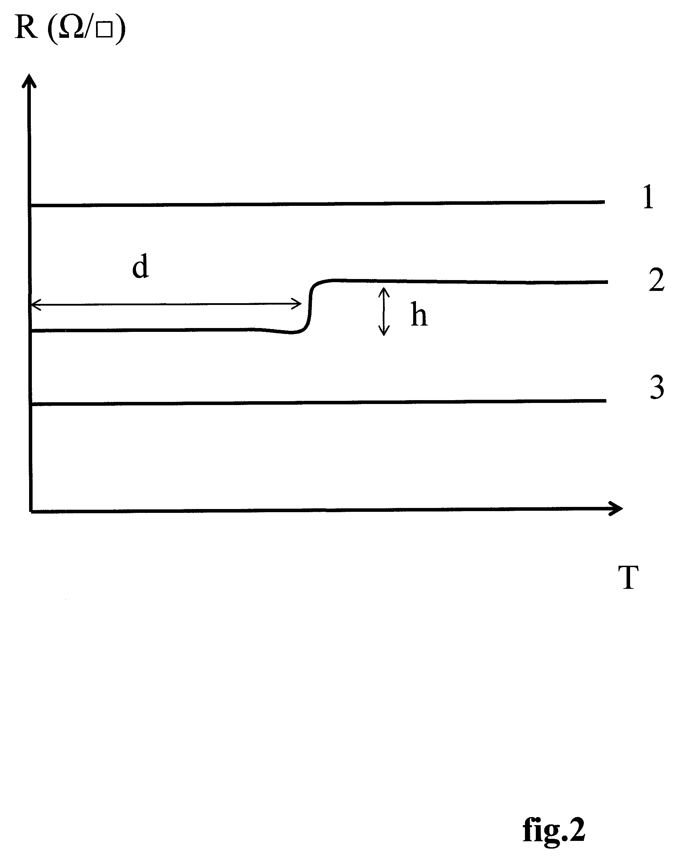

La figure 2 représente les évolutions temporelles de la résistivité superficielle en milieu ambiant:

1) Du matériau organique brut qui est par nature fortement isolant (courbe 1);
2) Du même matériau organique traité selon le procédé de l'invention pour le doter d'un réservoir de radicaux libres optimum facilement identifiable par un saut résistif (h) qui survient au bout d'un délai (d) (courbe 2). Le délai correspond au temps de diffusion de l'oxygène ambiant au travers de la couche de carbone amorphe (1). Le délai est d'autant plus important que cette couche est épaisse.
3) Du même matériau organique traité avec une dose plus forte produisant par réticulation une couche de carbone amorphe épaisse, de faible résistivité et extrêmement stable dans le temps (courbe 3).

L'axe des abscisses (T) représente le temps et l'axe des ordonnées (R) la résistivité superficielle exprimée en $\Omega/\square$.

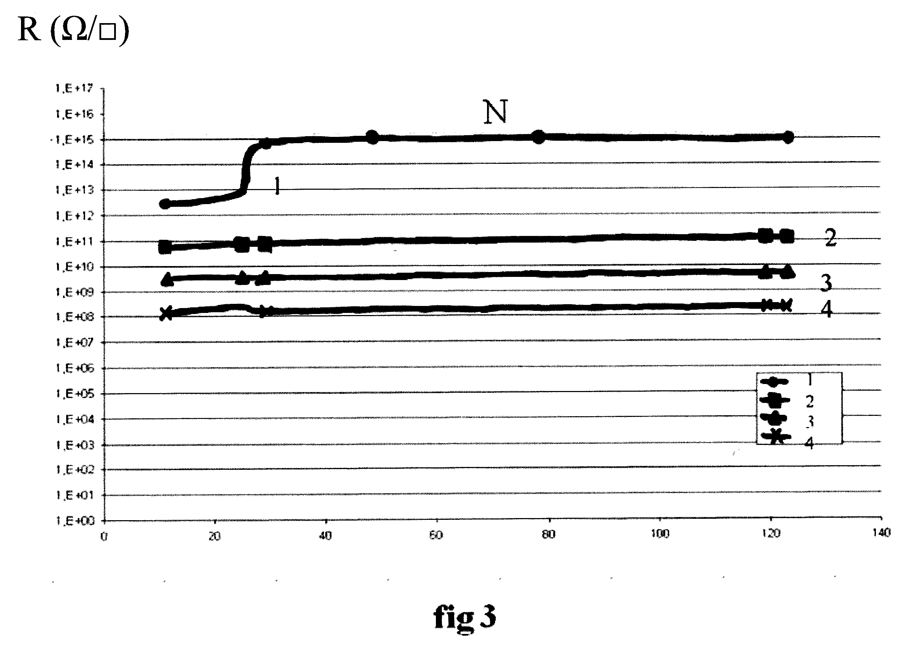

La figure 3 représente l'évolution expérimentale de la résistivité superficielle d'un polycarbonate en fonction du temps, pour différentes doses d'hélium égales à $10^{15}$ (courbe 1), $2,5.10^{15}$ (courbe 2), $5.10^{15}$ ions/cm² (courbe 3), $2,5.10^{16}$ ions/cm² (courbe 4). La mesure de résistivité a été effectuée selon la norme CEI 60093. La méthode pour mesurer de résistivité superficielle ne permet pas de mesurer des résistivités supérieures à $10^{15}\,\Omega/\square$. Ceci est représenté par la zone N située sur le graphique au dessus de $10^{15}\,\Omega/\square$. L'axe des abscisses représente le temps, exprimé en jours, séparant le traitement de l'échantillon à la mesure de sa résistivité superficielle. L'axe des ordonnées représente la mesure de la résistivité superficielle exprimée en $\Omega/\square$. Pour la courbe 1 associée à une dose de $10^{15}$ ions/cm², on constate qu'après traitement par le procédé de l'invention la résistivité superficielle diminue pendant un mois d'environs 3 ordres de grandeur, passant de $1,5.10^{16}\,\Omega/\square$ à $5.10^{12}\,\Omega/\square$, puis retrouve brutalement sa valeur d'origine située au alentour de $1,5.10^{16}\,\Omega/\square$. On distingue nettement un saut résistif de 3 ordres de grandeur autour de 30 jours se présentant sous la forme d'une marche. Ce saut résistif révèle l'existence d'un réservoir de radicaux libres en couche profonde qui se recombinent très rapidement avec l'oxygène de l'air. Sans vouloir être lié par une quelconque théorie scientifique, cette période de 30 jours doit représenter le temps de diffusion de l'oxygène ambiant au travers d'une couche de carbones relativement amorphes située en extrême surface et s'interposant entre le milieu ambiant et le réservoir de radicaux libres. Pour les courbes 2, 3 et 4 associées aux doses $2,5.10^{15}$, $5.10^{15}$, $2,5.10^{16}$ ions/cm², on constate que la résistivité superficielle reste constante sur plus de 120 jours autour de valeurs égales à $10^{11}\,\Omega/\square$ à $5.10^9\,\Omega/\square$ et $1,5.10^8\,\Omega/\square$. Sans vouloir être lié par une quelconque théorie scientifique, on suppose que les couches obtenues avec des doses supérieures à $2,5.10^{15}$ ions/cm² sont extrêmes stables, qu'elle comporte très peu de radicaux libres. Ces couches sont le fruit d'une réticulation totale aboutissant à la formation d'une couche de carbones amorphes. La mesure de résistivité superficielle est une méthode efficace pour identifier la dose, en l'occurrence $10^{15}$ ions/cm², permet un greffage optimal de monomères en couche profonde. Le procédé de l'invention préconise en règle générale d'identifier la dose pour laquelle le saut résistif est le plus élevé. Pour accélérer ce processus d'identification on peut augmenter la température des échantillons de manière à accroitre la vitesse de diffusion de l'oxygène ambiant.

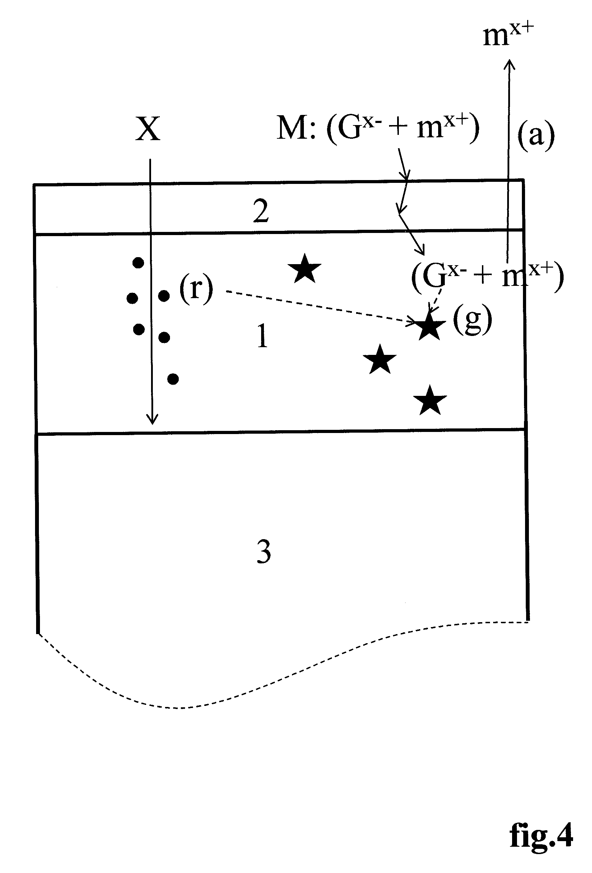

La figure 4 représente un mode de réalisation pour créer une couche antibactérienne consistant à bombarder le polymère avec des ions (X) pour créer un réservoir de radicaux libre (1) où s'effectue le greffage du monomère (M) par immersion dans une seule solution de monomères (M). Le monomère (M) comprend une partie greffable ($G^{x-}$) et un ion métallique bactéricide ($m^{x+}$) faiblement lié à ($G^{x-}$). Une fois le greffage effectué l'ion métallique bactéricide ($m^{x+}$) stocké peut se libérer dans une étape (a) au travers de la couche stabilisante (2) pour exercer son action bactéricide. On peut prendre comme exemple l'acrylate d'argent ($CH_2=CH\text{-}COO^{-}+Ag^{+}$).

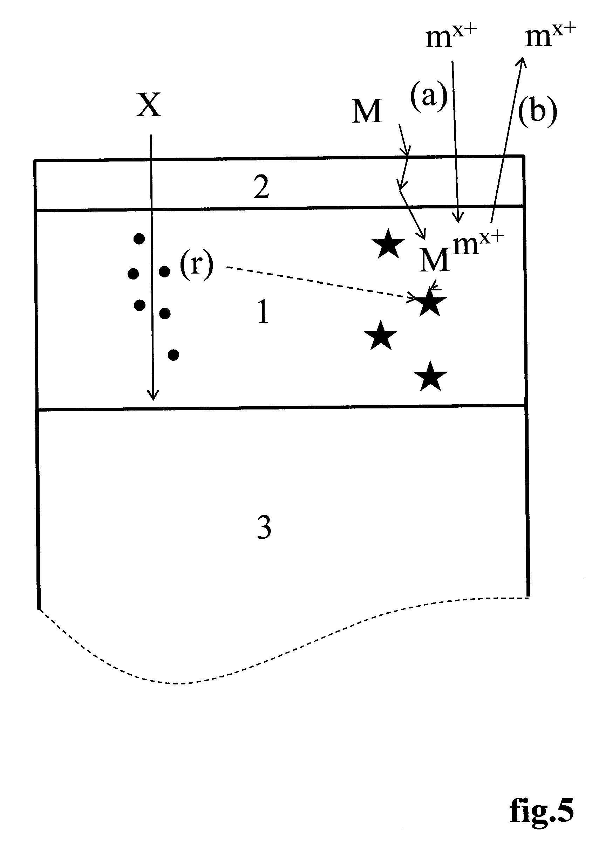

La figure 5 représente un second mode de réalisation pour créer une couche antibactérienne comprenant une première étape où le polymère est bombardé avec des ions (X) pour créer un réservoir de radicaux libres (1) ou s'effectue le greffage du monomère (M) par immersion dans une solution contenant ces monomères (M), puis une seconde étape où le polymère greffé est immergé dans une solution d'ions métalliques bactéricides ($m^{x+}$) qui diffusent dans une sous étape (a) au travers de la couche stabilisante (2) pour être stockés et fixés faiblement (chélation) aux monomères (M) de la couche (1) pour pouvoir diffuser à nouveau dans une sous étape (b) au travers de la couche stabilisante (2) pour exercer leur action bactéricide. On peut prendre comme exemple un greffage dans une solution d'acide acrylique et un stockage d'ions $Cu^{2+}$ provenant d'une immersion dans une solution de sulfate de cuivre.

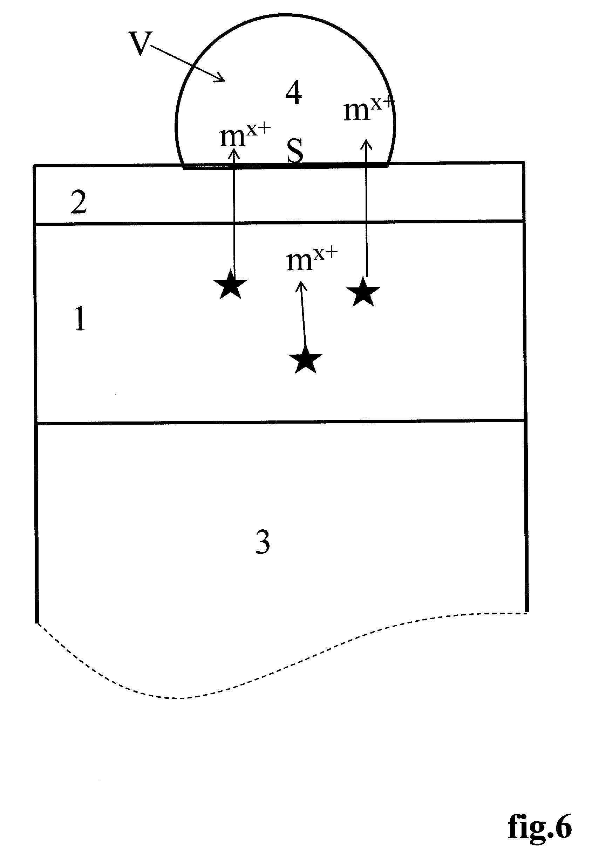

La figure 6 représente la libération d'ions métalliques bactéricides ($m^{x+}$) stockés dans la couche greffée (1), vers le fluide déposé sous forme de goutte (4) sur la surface de la couche (2). L'effet antibactérien est efficace lorsque la quantité d'ions métalliques diffusés dans le fluide dépasse un seuil de concentration bactéricide que l'on estime à 20 ppm (20 $\mu$g/cm$^3$) pour les ions $Ag^+$, à 10 ppm (10 $\mu$g/cm$^3$) pour les ions cuivre ($Cu^{2+}$). Pour un volume de fluide identique (V), la rapidité de diffusion peut évoluer en fonction de la surface de contact (S) : plus la surface est hydrophile, plus la surface de contact est étalée, plus rapide est la diffusion des ions métallique bactéricide dans le fluide. La concentration d'ions métalliques bactéricides maximale qui diffuse dans le volume du fluide est égale à (CsxS/V) ou Cs est la charge surfacique en ions métalliques bactéricides de la surface antibactérienne. Il est impossible, compte tenu des profondeurs de greffage obtenues pour des tensions d'accélération de 1000 kV de stocker plus 1000 $\mu$g/cm$^2$.

Selon différents modes de réalisation du procédé selon la présente invention, qui peuvent être combinés entre eux :

- le matériau organique est mobile par rapport au faisceau d'ions à une vitesse, $V_D$, comprise entre 0,1 mm/s et 1000 mm/s. Il est ainsi possible de déplacer l'échantillon pour traiter des zones dont la dimension est supérieure à celle du faisceau. La vitesse de défilement $V_D$, peut être constante ou variable. Selon un mode de réalisation, le matériau organique se déplace et le faisceau d'ions est fixe. Selon un autre mode de réalisation, le faisceau d'ions balaie le matériau organique. Il est également possible que le matériau organique se déplace quand le faisceau d'ions est mobile. Selon un mode de réalisation, une même zone du matériau organique est déplacée sous le faisceau d'ions selon une pluralité, N, de passages à la vitesse $V_D$. Il est ainsi possible de traiter une même zone du matériau organique avec une dose d'ions correspondant à la somme des doses d'ions reçues par cette zone à l'issue des N passages. On note également que si la taille du matériau organique le permet, l'étape de traitement peut être statique et résulter d'un ou plusieurs « flash » d'ions ;
- après traitement par faisceau d'ions le matériau organique est remis à l'air libre avant d'être plongée dans un liquide ou une atmosphère gazeuse contenant les monomères à greffer en couche profonde. Le laps de temps séparant le traitement par faisceau d'ions et l'immersion doit être le plus court possible pour éviter les recombinaisons avec l'oxygène et l'humidité ambiants. On choisira la température d'immersion de manière à ce que la vitesse de diffusion des monomères soit compatible avec la vitesse de défilement sous faisceau d'ions et qu'elle n'induise pas une modification des propriétés du matériau organique lors de son retour à la température ambiante.

Diverses modifications sont également possibles pour un homme du métier sans sortir du cadre de la présente invention tel que défini par les revendications annexées.

## Revendications

1. Procédé de traitement de surface d'un dispositif de distribution de produit fluide, **caractérisé en ce que** ledit procédé comprend l'étape de modifier par implantation ionique, au moyen de faisceaux d'ions multichargés et multi-énergies, au moins une surface à traiter d'au moins une partie dudit dispositif en contact avec ledit produit fluide, ladite surface modifiée ayant des propriétés limitant l'apparition et/ou la prolifération de bactéries sur ladite surface modifiée, lesdits ions multichargés étant choisis parmi l'hélium (He), le bore (B), le carbone (C), l'azote (N), l'oxygène (O), le néon (Ne), l'argon (Ar), le krypton (Kr), le xénon (Xe) l'implantation ionique étant réalisée à une profondeur de 0 à 3 $\mu$m, ledit procédé comprend un procédé de greffage de monomères en couche profonde dans un matériau organique, comprenant deux étapes successives :

   a) une étape de bombardement ionique par un faisceau d'ions pour créer :

   - un réservoir de radicaux libres dans une couche (1) d'une épaisseur $e_{rad}$ comprise entre 20 nm à 3000 nm;
   - une couche stabilisante (2) s'interposant entre la surface et le réservoir de radicaux libres (1) d'une épaisseur $e_{stab}$ comprise entre 0 nm à 3000 nm;
   - les ions du faisceau d'ions sont sélectionnés parmi les ions des éléments de la liste constituée de l'hélium (He), du bore (B), du carbone (C), de l'azote (N), de l'oxygène (O), du néon (Ne), de l'argon (Ar), du krypton (Kr), du xénon (Xe) ;
   - la tension d'accélération des ions est supérieure ou égale à 10 kV et inférieure ou égale à 1000 kV ;
   - la température de traitement du matériau organique est inférieure ou égale à sa température de fusion;

- on choisit la dose d'ions par unité de surface dans une plage comprise entre $10^{12}$ ions/cm$^2$ et $10^{18}$ ions/cm$^2$ en identifiant par une mesure de l'évolution temporelle de la résistivité superficielle du matériau organique la dose qui induit le saut de marche résistif le plus élevé ;

b) une étape de greffage de monomères consistant à diffuser des monomères (M) au travers d'une couche stabilisante (2) de la surface vers le réservoir de radicaux libres (1) à une température de diffusion $T_d$, la température de diffusion $T_d$ étant comprise entre la température ambiante et la température de fusion $T_f$ du matériau organique.

2. Procédé selon la revendication 1, dans lequel ledit faisceau d'ions est créé par une source à résonance cyclotronique électronique (RCE).

3. Procédé selon l'une quelconque des revendications précédentes, dans lequel lesdits ions multi-énergies sont implantés simultanément avec la même tension d'extraction.

4. Procédé selon l'une quelconque des revendications précédentes, dans lequel ladite au moins une surface à traiter est en matériau synthétique, comprenant notamment du polyéthylène (PE) et/ou de polypropylène (PP) et/ou du polychlorure de vinyle (PVC) et/ou du polytétrafluoroéthylène (PTFE).

5. Procédé selon l'une quelconque des revendications précédentes, dans lequel ladite au moins une surface à traiter est en élastomère.

6. Procédé selon l'une quelconque des revendications précédentes, dans lequel ledit dispositif de distribution comporte un réservoir contenant le produit fluide, un organe de distribution, tel qu'une pompe ou une valve, fixé sur ledit réservoir, et une tête de distribution pourvue d'un orifice de distribution, pour actionner ledit organe de distribution.

7. Procédé selon l'une quelconque des revendications précédentes, dans lequel ledit produit fluide est un produit fluide pharmaceutique destiné à être pulvérisé et/ou inhalé de manière nasale ou orale.

8. Procédé selon l'une quelconque des revendications précédentes, dans lequel ledit procédé est réalisé en continu sur la chaîne de montage et d'assemblage du dispositif de distribution de produit fluide.

9. Procédé selon l'une quelconque des revendications précédentes, dans lequel ladite étape de greffage est suivie d'une étape d'immersion dans une solution contenant des ions bactéricides.

10. Procédé selon l'une quelconque des revendications précédentes , dans lequel, pour un ion quelconque, l'étape du choix de la dose d'ions par unité de surface de manière à créer une couche stabilisante (2) et un réservoir de radicaux libres (1) est effectuée en s'appuyant sur des données expérimentales préalablement obtenues indiquant pour un autre type d'ion à une énergie données la dose d'ions par unité de surface permettant d'obtenir le saut de marche résistif le plus élevée.

11. Procédé selon l'une quelconque des revendications précédentes, dans lequel la dose d'ions par unité de surface est de préférence comprise entre $10^{13}$ ions/cm$^2$ et $5.10^{17}$ ions/cm$^2$.

12. Procédé selon l'une quelconque des revendications précédentes, dans lequel la tension d'accélération des ions est de préférence comprise entre 20 kV et 200 kV.

13. Procédé selon l'une quelconque des revendications précédentes, dans lequel les monomères (M) choisis présentent des propriétés hydrophiles et/ou hydrophobes et/ou anti bactériennes.

14. Procédé selon la revendication 13, dans lequel, pour un ion donné, l'étape du choix de l'énergie de manière à créer une charge surfacique d'ions métalliques bactéricides, stockés dans la couche greffée correspondant au réservoir de radicaux libre (1), permettant de dépasser le seuil de concentration bactéricide spécifique aux ions métalliques bactéricides, dans un fluide (4) de volume (V) et de surface de contact (S), est effectuée en s'appuyant sur des données préalablement établies permettant de représenter l'évolution du nombre d'ions métalliques bactéricides par unité de surface en fonction de l'épaisseur du traitement, de la densité volumique du polymère, de la masse molaire du monomère constituant le polymère, du nombre de monomères greffés par monomère constituant le polymère, du nombre d'ions métalliques bactéricides liés par monomère greffé.

**15.** Procédé selon l'une quelconque des revendications précédentes, dans lequel le matériau organique est mobile par rapport au faisceau d'ions à une vitesse, $V_D$, comprise entre 0,1 mm/s et 1000 mm/s.

**16.** Procédé selon la revendication 15, dans lequel une même zone du matériau organique est déplacée sous le faisceau d'ions selon une pluralité, N, de passages à la vitesse $V_D$.

**17.** Procédé selon l'une quelconque des revendications précédentes, dans lequel le matériau organique est choisit parmi la liste des matériaux appartenant à la famille des polymères, des élastomères ou des résines.

**Patentansprüche**

**1.** Verfahren zur Behandlung der Oberfläche einer Ausgabevorrichtung für ein fluides Produkt, **dadurch gekennzeichnet, dass** das Verfahren den Schritt des Modifizierens mindestens einer zu behandelnden Oberfläche mindestens eines Teils der Vorrichtung in Kontakt mit dem fluiden Produkt durch Ioneneinpflanzung mittels mehrfach geladenen Multi-Energie-Ionenstrahlen umfasst, wobei die modifizierte Oberfläche Eigenschaften hat, die das Auftreten und/oder die Ausbreitung von Bakterien auf der modifizierten Oberfläche begrenzt, wobei die mehrfach geladenen Ionen ausgewählt sind aus Helium (He), Bor (B), Kohlenstoff (C), Stickstoff (N), Sauerstoff (O), Neon (Ne), Argon (Ar), Krypton (Kr), Xenon (Xe), wobei die Ioneneinpflanzung in einer Tiefe von 0 bis 3 $\mu$m vorgenommen wird, wobei das Verfahren ein Monomer-Aufpropfverfahren in einer tiefen Schicht in einem organischen Material umfasst, umfassend die folgenden aufeinander folgenden Schritte:

a) einen Schritt des Ionenbeschusses durch einen Ionenstrahl um:

- ein Reservoir von freien Radikalen in einer Schicht (1) mit einer Dicke $e_{rad}$ zwischen 20 nm und 3000 nm zu schaffen;
- eine zwischen der Oberfläche und dem Reservoir von freien Radikalen (1) eingefügte stabilisierende Schicht (2) mit einer Dicke $e_{stab}$ zwischen 0 nm und 3000 nm zu schaffen;
- wobei die Ionen des Ionenstrahls aus den Ionen der Elemente der Liste bestehend aus Helium (He), Bor (B), Kohlenstoff (C), Stickstoff (N), Sauerstoff (O), Neon (Ne), Argon (Ar), Krypton (Kr), Xenon (Xe) ausgewählt sind;
- wobei die Beschleunigungsspannung der Ionen größer oder gleich 10 kV und kleiner oder gleich 1000 kV ist;
- wobei die Behandlungstemperatur des organischen Materials kleiner oder gleich seiner Schmelztemperatur ist;
- wobei die Dosis der Ionen pro Flächeneinheit in einem Bereich zwischen $10^{12}$ Ionen/cm$^2$ und $10^{18}$ Ionen/cm$^2$ ausgewählt wird, wobei die Dosis durch ein Maß der zeitlichen Entwicklung des spezifischen Oberflächenwiderstands des organischen Materials ermittelt wird, wodurch der größte Sprung im Widerstandsverlauf bewirkt wird;

b) einen Schritt des Monomer-Aufpropfens, der darin besteht, Monomere (M) über eine stabilisierende Schicht (2) der Oberfläche in Richtung zum Reservoir der freien Radikalen (1) bei einer Diffusionstemperatur $T_d$ zu diffundieren, wobei die Diffusionstemperatur $T_d$ zwischen der Umgebungstemperatur und der Schmelztemperatur $T_f$ des organischen Materials liegt.

**2.** Verfahren nach Anspruch 1, wobei der Ionenstrahl durch eine Elektron-Zyklotron-Resonanzquelle (EZR) erzeugt wird.

**3.** Verfahren nach einem der vorhergehenden Ansprüche, wobei die Multi-Energie-Ionen gleichzeitig mit der gleichen Extraktionsspannung eingepflanzt werden.

**4.** Verfahren nach einem der vorhergehenden Ansprüche, wobei die mindestens eine zu behandelnde Oberfläche aus einem synthetischen Material besteht, insbesondere aufweisend Polyethylen (PE) und/oder Polypropylen (PP) und/oder Polyvinylchlorid (PVC) und/oder Polytetrafluorethylen (PTFE).

**5.** Verfahren nach einem der vorhergehenden Ansprüche, wobei die mindestens eine zu behandelnde Oberfläche aus Elastomer besteht.

**6.** Verfahren nach einem der vorhergehenden Ansprüche, wobei die Ausgabevorrichtung einen Behälter, der das fluide Produkt enthält, eine Ausgabeeinrichtung, wie eine Pumpe oder ein Ventil, die auf dem Behälter befestigt ist, und einen Ausgabekopf, der mit einer Ausgabeöffnung versehen ist, um die Ausgabeeinrichtung zu betätigen, aufweist.

7. Verfahren nach einem der vorhergehenden Ansprüche, wobei das fluide Produkt ein pharmazeutisches fluides Produkt ist, welches dazu gedacht ist, zerstäubt und/oder nasal oder oral inhaliert zu werden.

8. Verfahren nach einem der vorhergehenden Ansprüche, wobei das Verfahren kontinuierlich an der Montage- und Zusammenbaustraße der Ausgabevorrichtung für ein fluides Produkt ausgeführt wird.

9. Verfahren nach einem der vorhergehenden Ansprüche, wobei auf den Aufpfropfschritt ein Schritt des Eintauchens in eine Lösung, die bakterizide Ionen enthält, umfasst.

10. Verfahren nach einem der vorhergehenden Ansprüche, wobei für irgendein Ion der Schritt der Auswahl der Dosis der Ionen pro Flächeneinheit, um eine stabilisierende Schicht (2) und ein Reservoir für freie Radikale (1) zu bilden, auf Basis von im Vorfeld erhaltenen Versuchsdaten erfolgt, die für eine andere Art von Ion einer gegebenen Energie die Ionendosis pro Flächeneinheit anzeigen, mit der der größte Sprung im Widerstandsverlauf erhalten werden kann.

11. Verfahren nach einem der vorhergehenden Ansprüche, wobei die Ionendosis pro Flächeneinheit vorzugsweise zwischen $10^{13}$ Ionen/cm$^2$ und $5.10^{17}$ Ionen/cm$^2$ liegt.

12. Verfahren nach einem der vorhergehenden Ansprüche, wobei die Beschleunigungsspannung der Ionen vorzugsweise zwischen 20 kV und 200 kV liegt.

13. Verfahren nach einem der vorhergehenden Ansprüche, wobei die ausgewählten Monomere (M) hydrophile und/oder hydrophobe und/oder antibakterielle Eigenschaften aufweisen.

14. Verfahren nach Anspruch 13, wobei für ein gegebenes Ion der Schritt der Auswahl der Energie, um eine Oberflächenladung von metallischen bakteriziden Ionen zu schaffen, die in der aufgepfropften Schicht gelagert sind, die dem Reservoir von freien Radikalen (1) entspricht, der es ermöglicht, die bakterielle Konzentrationsschwelle, die für die metallischen bakteriellen Ionen spezifisch ist, in einem Fluid (4) von einem Volumen (V) und einer Kontaktfläche (S) zu überschreiten, auf Basis von im Vorfeld ermittelten Daten erfolgt, die die Entwicklung der Zahl von metallischen bakteriziden Ionen pro Flächeneinheit in Abhängigkeit von der Behandlungsdicke, der Volumendichte des Polymers, der molaren Masse des das Polymer bildenden Monomers, der Anzahl der aufgepfropften Monomere pro das Polymer bildende Monomer, der Anzahl der metallischen bakteriziden Monomere, die durch das aufgepfropfte Monomer gebunden sind, darstellen können.

15. Verfahren nach einem der vorhergehenden Ansprüche, wobei das organische Material in Bezug auf den Ionenstrahl mit einer Geschwindigkeit $V_D$ zwischen 0,1 mm/s und 1000 mm/s beweglich ist.

16. Verfahren nach Anspruch 15, wobei ein gleicher Bereich des organischen Materials unter dem Ionenstrahl nach einer Vielzahl, N, von Durchgängen mit der Geschwindigkeit $V_D$ verlagert wird.

17. Verfahren nach einem der vorhergehenden Ansprüche, wobei das organische Material aus der Liste von Materialien, die zur Familie der Polymere, Elastomere oder Harze gehört, ausgewählt wird.

**Claims**

1. A method of surface treating a fluid dispenser device, **characterized in that** said method comprises a step of modifying, by ionic implantation, by means of multi-charged and multi-energy ion beams, at least one surface to be treated of at least a portion of said device in contact with said fluid, said modified surface having properties limiting the appearance and/or proliferation of bacteria on said modified surface, said multi-charged ions being selected from helium (He), boron (B), carbon (C), nitrogen (N), oxygen (O), neon (Ne), argon (Ar), krypton (Kr), and xenon (Xe),ionic implantation being carried out to a depth of 0 $\mu$m to 3 $\mu$m, said method comprising a method of deep layer grafting monomers into an organic material, comprising two steps in succession:

a) a step of ionic bombardment by an ion beam to create:

- a reservoir of free radicals in a layer (1) with a thickness $e_{rad}$ in the range 20 nm to 3000 nm; and
- a stabilizing layer (2) interposed between the surface and the reservoir of free radicals (1) with a thickness $e_{stab}$ in the range 0 nm to 3000 nm;
- the ions of the ion beam are selected from the ions of elements in the list constituted by helium (He), boron (B), carbon (C), nitrogen (N), oxygen (O), neon (Ne), argon (Ar), krypton (Kr), and xenon (Xe);
- the ion acceleration voltage is greater than or equal to 10 kV and less than or equal to 1000 kV; and
- the treatment temperature of the organic material is less than or equal to its melting temperature;
- the ion dose per unit area is selected so as to be in the range $10^{12}$ ions/cm² to $10^{18}$ ions/cm² by using a measurement of the change over time of the surface resistivity of the organic material to identify the dose that induces the greatest resistive jump step;

b) a step of grafting monomers, consisting of diffusing monomers (M) through a stabilizing layer (2) from the surface towards the reservoir of free radicals (1) at a diffusion temperature $T_d$, the diffusion temperature $T_d$ being in the range from ambient temperature to the melting temperature $T_f$ of the organic material.

2. A method according to claim 1, wherein said ion beam is created by an electron cyclotron resonance (ECR) source.

3. A method according to either preceding claim, wherein said multi-energy ions are implanted simultaneously with the same extraction voltage.

4. A method according to any preceding claim, wherein said at least one surface to be treated is formed from synthetic material, in particular comprising polyethylene (PE) and/or polypropylene (PP) and/or polyvinyl chloride (PVC) and/or polytetrafluoroethylene (PTFE).

5. A method according to any preceding claim, wherein said at least one surface to be treated is formed from an elastomer.

6. A method according to any preceding claim, wherein said dispenser device comprises a reservoir containing the fluid, a dispenser member such as a pump or a valve attached to said reservoir, and a dispenser head provided with a dispenser orifice in order to actuate said dispenser member.

7. A method according to any preceding claim, wherein said fluid is a pharmaceutical fluid for spraying and/or inhaling nasally or orally.

8. A method according to any preceding claim, wherein said method is carried out continuously on an assembly line for the fluid dispenser device.

9. A method according to any preceding claim, wherein said grafting step is followed by a step of immersion in a solution containing bactericidal ions.

10. A method according to any preceding claim, wherein for any ion, the step of selecting the dose of ions per unit area so as to create a stabilizing layer (2) and a reservoir of free radicals (1) is carried out on the basis of experimental data that have already been obtained indicating, for another type of ion at a given energy, the dose of ions per unit area that can produce the highest resistive jump step.

11. A method according to any preceding claim, wherein the dose of ions per unit area is preferably in the range $10^{13}$ ions/cm² to $5 \times 10^{17}$ ions/cm².

12. A method according to any preceding claim, wherein the ion acceleration voltage is preferably in the range 20 kV to 200 kV.

13. A method according to any preceding claim, wherein the monomers (M) that are selected have hydrophilic and/or hydrophobic and/or antibacterial properties.

14. A method according to claim 13, wherein for a given ion, the step of selecting the energy so as to create a surface loading of bactericidal metal ions stored in the grafted layer corresponding to the reservoir of free radicals (1) allowing a threshold bactericidal concentration specific to the bactericidal metal ions to be exceeded in a fluid (4) with volume (V) and contact surface area (S) is carried out on the basis of data that have already been established that can be used to represent the change in the number of bactericidal metal ions per unit area as a function of the thickness of the treatment, the bulk density of the polymer, the molar mass of the monomer constituting the polymer, the number of grafted monomers per monomer constituting the polymer, and the number of bactericidal metal ions bonded by the grafted monomer.

15. A method according to any preceding claim, wherein the organic material is movable relative to the ion beam at a speed $V_D$ in the range 0.1 mm/s to 1000 mm/s.

16. A method according to claim 15, wherein the same zone of organic material is moved beneath the ion beam in a plurality, N, of passes at the speed $V_D$.

17. A method according to any one of claims 9 to 18, wherein the organic material is selected from the list of materials belonging to the family of polymers, elastomers, or resins.

fig.1

fig.2

R (Ω/□)

**fig 3**

fig.4

fig.5

**fig.6**

**EP 2 588 639 B1**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 0473892 A
- EP 0580460 A
- EP 0831972 A
- US 6227413 B
- EP 1169241 A
- DE 2830977
- US 5154325 A
- EP 0644785 A
- US 5433343 A
- EP 0889757 A
- WO 2010100384 A
- WO 2008141141 A
- WO 2005085491 A

**Littérature non-brevet citée dans la description**

- **J.F. ZIEGLER.** The Stopping and Range of Ions in Matter. Pergamon Press, 1977, vol. 2-6
- **J.F. ZIEGLER ; J.P. BIERSACK ; U. LITTMARK.** The Stopping and Range of Ions in Solids. Pergamon Press, 1985
- **J.P. BIERSACK ; L. HAGGMARK.** *Nucl. Instr. and Meth.,* 1980, vol. 174, 257